(19) **Europäisches Patentamt / European Patent Office / Office européen des brevets**

(11) **EP 2 870 477 B1**

(12) # EUROPÄISCHE PATENTSCHRIFT

(45) Veröffentlichungstag und Bekanntmachung des Hinweises auf die Patenterteilung:
**27.12.2017   Patentblatt 2017/52**

(21) Anmeldenummer: **13765281.4**

(22) Anmeldetag: **09.07.2013**

(51) Int Cl.:
*G01N 33/558* (2006.01)        *G01N 33/573* (2006.01)
*G01N 33/574* (2006.01)        *G01N 33/72* (2006.01)

(86) Internationale Anmeldenummer:
**PCT/DE2013/000380**

(87) Internationale Veröffentlichungsnummer:
**WO 2014/008884 (16.01.2014 Gazette 2014/03)**

(54) **TESTKIT (KOMBI-SCHNELLTEST) ZUM SYNCHRONEN NACHWEIS VON BIOMARKERN IM STUHL ZUR ERKENNUNG PATHOLOGISCHER VERÄNDERUNGEN IM GASTROINTENSTINALTRAKT, INSBESONDERE IM DARM**

TEST KIT (COMBINATION QUICK TEST) FOR SYNCHRONOUS DETECTION OF BIOMARKERS IN FECES FOR IDENTIFICATION OF PATHOLOGICAL CHANGES IN THE GASTROINTESTINAL TRACT, ESPECIALLY IN THE INTESTINE

TROUSSES D'ANALYSE (TROUSSES D'ANALYSE RAPIDE COMBINATOIRE) POUR UNE DÉTECTION SYNCHRONE DE BIOMARQUEURS POUR L'IDENTIFICATION DE CHANGEMENTS PATHOLOGIQUES DE SYSTEME GASTROINTESTINAL, EN PARTICULIER DANS L'INTESTIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2012   DE 102012013888**
**18.12.2012   DE 202012012084 U**

(43) Veröffentlichungstag der Anmeldung:
**13.05.2015   Patentblatt 2015/20**

(73) Patentinhaber: **ScheBo Biotech AG**
**35394 Giessen (DE)**

(72) Erfinder:
• **SCHEEFERS, Hans**
**35435 Wettenberg (DE)**
• **SCHEEFERS-BORCHEL, Ursula**
**35435 Wettenberg (DE)**

(74) Vertreter: **Jungblut & Seuss Patentanwälte**
**Max-Dohrn-Strasse 10**
**10589 Berlin (DE)**

(56) Entgegenhaltungen:
EP-A2- 0 560 411          WO-A1-2005/005991
WO-A1-2007/071366         WO-A2-01/21826
US-A1- 2006 134 804

• **CAROLIN TONUS ET AL: "The faecal tumour M2-PK screening test for invasive & pre-invasive colorectal cancer: estimated specificity & results as a function of age for a study population of 4854 volunteers", JOURNAL OF ONCOLOGY, Bd. 59, 1. Januar 2009 (2009-01-01), Seiten 32e-37e, XP055090399,**
• **TROJAN J ET AL: "A NEW IMMUNOLOGICAL TEST STRIP DEVICE FOR THE RAPID, QUALITATIVE DETECTION OF FAECAL OCCULT BLOOD", ZEITSCHRIFT FUER GASTROENTEROLOGIE, GEORG THIEME VERLAG, DE, Bd. 40, Nr. 11, 1. November 2002 (2002-11-01), Seiten 921-924, XP008033051, ISSN: 0044-2771, DOI: 10.1055/S-2002-35415**
• **J Blessing ET AL: "Verbesserte Darmkrebsvorsorge durch Stuhldiagnostik: M2-PK / Immunologischer Blutnachweis", , 1. Januar 2004 (2004-01-01), Seite 1, XP055090064, Gefunden im Internet: URL:http://www.labor-blessing.de/files/pub /M2PK_2004_Stand0104.pdf [gefunden am 2013-11-25]**
• **J Blessing ET AL: "Untersuchungsprogramm", , 17. April 2012 (2012-04-17), Seiten 1-428, XP055090069, Gefunden im Internet: URL:https://web.archive.org/web/2012041707 1105/http://www.labor-blessing.de/files/uv z/UVZ.pdf [gefunden am 2013-11-25]**

EP 2 870 477 B1

**(Forts. nächste Seite)**

- UCHIDA K ET AL: "Immunochemical detection of human blood in feces", CLINICA CHIMICA ACTA, ELSEVIER BV, AMSTERDAM, NL, Bd. 189, Nr. 3, 31. August 1990 (1990-08-31), Seiten 267-274, XP023396920, ISSN: 0009-8981, DOI: 10.1016/0009-8981(90)90308-F [gefunden am 1990-08-31]
- YOGESH M. SHASTRI ET AL: 'Comparison of an Established Simple Office-Based Immunological FOBT With Fecal Tumor Pyruvate Kinase Type M2 (M2-PK) for Colorectal Cancer Screening: Prospective Multicenter Study' AMERICAN JOURNAL OF GASTROENTEROLOGY Bd. 103, Nr. 6, 01 Juni 2008, US, Seiten 1496 - 1504, XP055278486 DOI: 10.1111/j.1572-0241.2008.01824.x ISSN: 0002-9270
- Anonym: "PreventID CC Weiterführende Informationen", , 1 January 2007 (2007-01-01), pages 1-7, XP055278489, Bensheim Retrieved from the Internet: URL:http://www.preventis-online.de/fileadm in/pdf-dateien/checks/preventid-cc [retrieved on 2016-06-07]
- Hermann Brenner ET AL: "Inter-test agreement and quantitative crossvalidation of immunochromatographical fecal occult blood tests", International Journal of Cancer, vol. 127, no. 7, 4 January 2010 (2010-01-04), pages 1643-1649, XP055278492, US ISSN: 0020-7136, DOI: 10.1002/ijc.25154

**Beschreibung**

**Gebiet der Erfindung**

Die Erfindung betrifft einen gewerblich anwendbaren, kostensenkenden neuen Testkit (Kombi-Schnelltestkassette), in-vitro-Medizinprodukt, lateral flow Kassette, point of care test, Kombi-Schnelltest, Bioassay, Rapid-Lateral-Flow-Test-Strips, platform), zur Durchführung eines Verfahrens zum Nachweis von Biomarkern im menschlichen Stuhl. Der Testkit dient der Gesundheitsanalyse, Früherkennung und "Darmkrebsvorsorge" durch den Nachweis von Biomarkern, die einen Hinweis auf pathologische Veränderungen insbesondere eines malignen Geschehens im Gastrointestinaltrakt (Speiseröhre, Magen, Dünndarm, Gallenwege, Bauchspeicheldrüse und Dickdarm), insbesondere Tumoren und Tumorvorstufen (Polypen, Adenomen) des Darmes geben können. Die Erfindung betrifft im Besonderen einen Kombi-Schnelltest und eine Kombi-Schnelltestkassette und deren Bestandteile sowie die nötigen Reagenzien zur synchronen Durchführung eines immunchromatographischen Nachweisverfahrens. Synchron in der sprachlichen Bedeutung von "gleichzeitig", aber auch synchron in der technischen Bedeutung der gemeinsamen, optimalen Abstimmung von Wirkpaarungen (der chromatographischen Bedingungen, Membranen, Reagenzien, Dispensierrate, usw. siehe Beschreibungen) zum Nachweis der beiden Biomarker Tumor M2-PK und Hämoglobin in einer Kombi-Schnelltestkassette.

Die Erfindung betrifft darüber hinaus ein einfacheres Verfahren zur Auswertung des Tests und der Darstellung der Ergebnisse ("Risiko-Ampel").

Der Kombi-Schnelltest dient der "Vorfilterung" von Probanden im Rahmen eines Darmkrebs-Screening-Programms. Da die Durchführung des nicht invasiven Kombi-Schnelltests wesentlich weniger Angst als die Durchführung einer invasiven Koloskopie beim Probanden auslöst, führt der Test zu einer erhöhten Teilnahme an der Koloskopie.

**Hintergrund der Erfindung**

Krebs, besonders Darmkrebs ist die häufigste Krebserkrankung in Deutschland (65.000 Neuerkrankungen, 27.000 Todesfälle) europaweit sterben 200.000 Menschen im Jahr an dieser tückischen Krankheit. Aufgrund der Entstehungsart von Darmkrebs sind die Folgen der Darmkrebserkrankungen durch ein erfolgreiches Screening, d.h. der Erkennung von Vorstufen (Polypen/Adenomen) und deren Entfernung stark minimierbar.

Alle 20 Minuten stirbt in Deutschland ein Mensch an Darmkrebs. Darmkrebs entwickelt sich fast immer aus zunächst gutartigen Wucherungen der Darmschleimhaut (Mukosa). Der Krebs wächst langsam und lange Zeit ohne dass der Betroffene etwas merkt. Beschwerden tauchen meist erst dann auf, wenn der Tumor groß ist bzw. schon Metastasen gebildet hat. Unbehandelt führt Darmkrebs dann häufig innerhalb von 12 Monaten zum Tod.

Aufgrund seiner Entstehungsart sind die Folgen von Darmkrebs durch ein erfolgreiches Screening von asymptomatischen Probanden fast vollständig vermeidbar. Vorstufen (Polypen/Adenome) lassen sich durch Screeningmaßnahmen früh erkennen. Koloskopie gilt als das beste Verfahren (Goldstandard) im Rahmen der Früherkennung von Darmkrebs. Die Koloskopie stellt ein invasives, nur von speziell ausgebildeten Ärzten (z.B. Gastroenterologen) durchführbares Verfahren dar. Dieses Verfahren ist mit Risiken (z.B. Performation, Risiken durch Sedierung) verbunden. Die Vorsorgekoloskopie wird allen Menschen ab 55 Jahren als Kassenleistung angeboten. Die Teilnahmerate liegt aber nur bei 2-3 % und ist trotz erheblicher Aufklärung und Marketingaktionen, "Glitzerweltaktionen" inzwischen sogar rückläufig! Der Grund hierfür liegt unter Anderem in der Prozedur der Untersuchung, die viele Menschen als unangenehm empfinden (psychologische Hemmschwelle).

Aufgabe der Erfindung:

1. Senkung der psychologischen Hemmschwelle.
2. Erhöhung der Teilnehmerrate an der Vorsorgekoloskopie
3. Kalibrierung (Aufheben von Qualitätsunterschieden)
4. Standardisierung
5. Erhöhung der diagnostischen Sicherheit

Lösungsweg: Die Bereitstellung eines einfachen, robusten, kostengünstigen, routinetauglichen Lateral-Flow-Tests, der zudem durch den synchronen Nachweis von zwei besonderen Biomarkern (M2-PK und Hämoglobin) zur Erkennung von krankhaften gastrointestinalen Erkrankungen insbesondere Darmkrebs und dessen Vorstufen geeignet ist. Der Kombi-Schnelltest, der einfach und problemlos durchführbar ist, ist geeignet, die psychologische Hemmschwelle gegenüber der Screening-Koloskopie zu senken. Schon die positive Befundung nur eines Biomarkers, M2-PK und/oder Haemoglobin, bewirkt die Maßnahme Koloskopie zwecks weiterer Befundung durch den Arzt. Dies führt zu einer höheren Erkennungsrate von Darmkrebs und dessen Vorstufen durch die Koloskopie, da mit dem vorgeschalteten Kombi-Schnelltest eine Vorauswahl der Probanden erfolgt. Da der Kombi-Schnelltest zwei wichtige Biomarker für Darmkrebs synchron bestimmt, dient dieser Test als zweifacher Filter vor der Koloskopie.

Darmkrebs mit seinen Vorstufen (Polyp-cancer-sequence) beginnt auf der Innenseite des Darmes (Mukosa) und weitet sich dann in anderen Gewebeareale (Submukosa, Muskularis, Serosa) aus. Überschreitet das Tumorwachstum die Schleimhautgrenze spricht man von einem Darmkrebs in frühem oder fortgeschrittenem Stadium. Da der Stuhl direkten Kontakt zur Mukosa des Darmes hat, sind Biomarker als Indikatoren für pathologische Veränderungen im Gastrointestinaltrakt früher und spezifischer analytisch nachweisbar als im Blut. Aus diesem Grund ist das Stuhlprobenmaterial dem Blutprobenmaterial vorzuziehen. Erst wenn der Tumor die Submukosa erreicht hat, sind frühe Biomarker im Blut nachzuweisen. Sind diese Marker im Blut nachweisbar, so handelt es sich häufig um "späte" Formen von Darmkrebs und dessen Vorstufen. Generell ist die Vermessung von Biomarkern im Stuhl vorteilhaft im Vergleich zur Messung von Biomarkern im Blut. Je früher ein Darmkrebs und dessen Vorstufen erkannt werden, desto besser ist die Prognose. Die eingeleitete Therapie (z.B. kurative Koloskopie, "kleine viscerale" Chirurgie) führt zu einer fast 100%igen Heilung bei Darmkrebs im Frühstadium.

Als "Goldstandard" zur Prüfung eines malignen, insbesondere tumorösen, Prozesses im Darm gilt die Koloskopie.

Nur speziell qualifizierte Ärzte dürfen die Koloskopie durchführen. Die Koloskopie hat weiterhin den Nachteil, dass sie nicht standardisiert ist. Auch werden die "falschen" Probanden koloskopiert. Bei der Darmspiegelung (Koloskopie), die ein zentraler Bestandteil der Früherkennung ist, sind rund 70 % der Befunde unauffällig. In nur ca. einem Viertel der Fälle werden Vorstufen und in 1% der Fälle ein Karzinom diagnostiziert. Die Vorsorge-Koloskopie ist deshalb ein sehr ineffizientes und zudem teures Screeningverfahren, da zu viele gesunde Probanden koloskopiert werden (Gesundheitskosten senken!).

Daher ist es wünschenswert einen nicht-invasiven, kostengünstigen und sensitiven, verlässlichen Test, der mehrere Biomarker synchron misst, der Koloskopie vorzuschalten, um Probanden mit einem positiven Testbefund gezielt der Koloskopie zuzuführen (Senkung der Mortalität). Durch den erfindungsgemäßen nicht-invasiven Kombi-Schnelltest lässt sich die Teilnahmerate an einem Screening-Programm zur Erkennung von Darmkrebs und dessen Vorstufen stark erhöhen und somit eine signifikanten Steigerung der Überlebensrate von Patienten (im Gegensatz zu Probanden!) mit Darmkrebs sowie dessen Vorstufen erreichen. Darmkrebs und dessen Vorstufen sofern sie rechtzeitig erkannt werden ergeben fast eine 100 %ige Heilungschance für den Patienten; Befundung: Erhöhte M2-PK-Werte und/oder Hämoglobin-Werte führt unter Zuhilfenahme von Anamnese usw. ein zur Auslösung eines "Bündels" von Maßnahmen und zu weiteren Schritten, die zur Diagnose Darmkrebs führen. Die Diagnose wiederum führt zu einer Handlungsanweisung des Arztes z.B. chirurgische Therapiemaßnahmen und diese wiederum führen zur Heilung von Darmkrebs. Z.B. Therapie durch Chirurgie kann die Letalität senken.

Wie bei allen diagnostischen Verfahren sind auch bei in-vitro-Tests die sog. Sensitivität und die sog. Spezifität die entscheidenden Qualitätsmerkmale. Die Sensitivität ist die Wahrscheinlichkeit, eine erkrankte Person als krank zu erkennen. Dieses Gütemaß ist medizinisch besonders wichtig, da bei einer Früherkennungsmaßnahme natürlich möglichst wenige erkrankte Menschen übersehen werden dürfen. Die Spezifität ist die Wahrscheinlichkeit, eine gesunde Person als gesund zu erkennen. Auch diese Maß ist bei der Früherkennung bedeutsam, da vermieden werden soll, dass gesunde Menschen, die fälschlicherweise als krank diagnostiziert werden, eine überflüssige Behandlung erhalten.

**Stand der Technik**

**Guajak-Test (gFOBT) (Probenmaterial: Stuhl)**

Dieser chemische Stuhltest funktioniert auf der Guajak-Harzbasis. Durch eine chemische Redox-Reaktion werden verborgene (okkulte) Blutmengen in den Stuhlproben der Patienten nachgewiesen. Da die Grundlage dieses chemischen Tests ein Redox-Potential ist und dieses Redox-Potential durch Oxidationsmittel und Reduktionsmittel gestört wird, kommt es zu Störungen des Testes und zu einer geringen Sensitivität und Spezifität. Die Oxidationsmittel und Reduktionsmittel sind in der Nahrung enthalten. Darum besitzt dieser Test eine geringe Sensitivität und Spezifität und gilt daher als überholt und veraltet. Zudem können nur stark blutende Formen, jedoch nicht schwach blutende Formen von Darmkrebs oder dessen Vorstufen nachgewiesen werden. Außerdem ist bekannt, dass es blutende, nicht-blutende und intermittierend blutende Formen von Darmkrebs und dessen Vorstufen gibt. Je nach Zeitpunkt der Probennahmen können folglich Darmkrebs und dessen Vorstufen übersehen werden.

**Immunologischer Stuhltest (iFOBT) (Probenmaterial: Stuhl)**

Immunologische in-vitro-Tests fahnden ebenfalls nach okkultem Blut, das durch Vorstufen (Polypen) oder Frühstadien in den Stuhl gelangt. Der Nachweis erfolgt durch den Einsatz spezifischer Antikörper gegen Hämoglobin, bzw. gegen den Hämoglobin/Haptoglobin-Komplex. Mehr als 10 Hersteller/Distributoren bieten diese iFOB-Tests an. Die im Markt angebotenen Tests weisen jedoch extreme Qualitätsunterschiede (gemäß Sensitivität und Spezifität) auf, diese Unterschiede können nur Spezialisten erkennen, nicht jedoch wie gewünscht die große Zahl der niedergelassenen Ärzte (Anwender).

Des Weiteren ist zu erwähnen, dass diese sogenannten Blut-im-Stuhl Tests nur indirekt Darmkrebs und dessen Vorstufen nachweisen, da sie nicht spezifisch für Krebs und dessen Vorstufen, sondern nur für den Nachweis von Blut (unspezifisch für Darmkrebs) sind. Diese Tests können wie auch die klassischen Guajak-Tests (gFOBT), nur blutende Formen von Darmkrebs oder dessen Vorstufen nachweisen. Zudem ist bekannt, dass es blutende, nicht-blutende und intermittierend blutende Formen von Darmkrebs und dessen Vorstufen gibt. Je nach Zeitpunkt der Messung können folglich Darmkrebs und dessen Vorstufen übersehen werden.

Mit dem gFOBT und dem iFOBT lassen sich nur blutende Tumoren und deren Vorstufen nachweisen.

Diese Tests "erkennen" und "übersehen" daher Darmkrebs und dessen Vorstufen.

**Generell:** Ursächlich und auch pathophysiologisch hat okkultes Blut im Stuhl daher nur indirekt mit Darmkrebs und dessen Vorstufen zu tun!

**Enzymatischer Stuhltest (M2-PK) (Probenmaterial: Stuhl)**

Hierbei handelt es sich um einen Test, der nicht okkultes Blut, sondern ein für Krebs typisches Enzym im Stuhl nachweisen kann. Das Enzym kommt immer in bösartig verändertem Gewebe von verschiedenen Krebsarten in größeren Mengen vor - darunter auch bei Darmkrebs oder bösartig veränderten Darmpolypen. Nachgewiesen wird bei diesem Test, der auch auf dem Markt im Format des lateral-flow-Tests und ELISA erhältlich ist, der Biomarker Tumor M2-PK. Die Tumor M2-PK ist ein spezielles Isoenzym der Pyruvatkinase (PK). Im Normalgewebe kommt die Pyruvatkinase (M1-, M2-PK-Form) als tetramere Form (M2-PK-tetramer) vor. Bei Krebs und dessen Vorstufen findet man immer vermehrt die dimere Form dieser PK **(M2-PK-dimer = Tumor M2-PK).** Gegen dieses spezielle Tumor M2-PK-Molekül sind zwei unterschiedliche monoklonale Antikörper generiert worden. Diese speziellen Tumor M2-PK spezifischen Antikörper werden in den auf dem Markt befindlichen Tests verwendet (eingesetzt im ELISA- und/oder lateral-flow-Format).

**Generell:** Ursächlich und auch pathophysiologisch hat der Biomarker M2-PK direkt mit Krebs und dessen Vorstufen zu tun. Das Enzym M2-PK (dimere Form des Isoenzyms M2-PK) ist bei allen bisher untersuchten Tumoren (z.B. 12 Tumoren unterschiedlicher Gewebe/Organe) immer nachweisbar, auch bei Darmkrebs und dessen Vorstufen.

Klinische Untersuchungen haben ergeben, dass die auf dem Markt befindlichen Tumor M2-PK Tests (im lateral-flow- und/oder ELISA-Format) sowohl blutenden als auch nicht blutenden Darmkrebs und dessen Vorstufen nachweisen können.

Diese Tests (Teststreifen) können aber auch Darmkrebs und dessen Vorstufen "übersehen". Deshalb ist es auch eine Aufgabe der vorliegenden Erfindung, bessere M2-PK-Tests (Teststreifen) anzubieten.

Technische Lösung: erfindungsgemäßer Testkit beinhaltend einen Kombi-Schnelltest bestehend aus einer Plastikkassette als auch zwei neuen, gemäß Stand der Technik besseren Teststreifen zur Bestimmung der M2-PK und des Hämoglobins ("iFOBT", "immunologischer Test", "Okkultbluttest").

**Bluttest (Probenmaterial: Blut)**

Tumoren geben DNA (und/oder methylierte DNA) in den Blutkreislauf ab. Darmkrebs hinterlässt auf diese Weise eine typische Signatur - einen Biomarker. Dieser Biomarker ist mit einem Bluttest nachweisbar. Der Arzt nimmt hierfür beim Probanden eine Blutprobe ab und schickt sie an ein Speziallabor zur relativ aufwendigen, teuren Messung und Auswertung mittels, für den Alltag nicht sehr robuster PCR-Technologie.

**Generell:** Ursächlich und auch pathophysiologisch hat ein DNA-Methylierungsmuster, eine Signatur, eine typische Spur (Biomarker SEPT9, Septin9-Bluttest) nur indirekt mit Krebs und dessen Vorstufen zu tun.

Mit den M2-PK-Tests lassen sich blutende und nicht-blutende Tumoren und dessen Vorstufen nachweisen. Die M2-PK-Tests "übersehen" aber auch Darmkrebs und dessen Vorstufen. Daher ist eine Aufgabe der vorliegenden Erfindung, eine Verbesserung zu erzielen. Dieses Aufgabe wird durch die Ansprüche und die technische Beschreibung der vorliegenden Erfindung gelöst.

Nachteil der bisher bekannten Tumor M2-PK Tests (im lateral-flow- PCT/EP00/09303 und/oder ELISA-Format) als auch des gFOBT und iFOBT ist, dass keiner der drei Tests, für sich genommen, den gesamten diagnostisch relevanten Zeitraum, insbesondere patientenbezogen abdeckt.

Aus der WO2007/071366A1 (Roche Diagnostics GmbH) ist ein Verfahren bekannt, bei dem sowohl Tumor M2-PK, als auch Hämoglobin im Stuhl bestimmt werden. Die quantitative Bestimmung beider Parameter erfolgt in zwei getrennten Tests, die Auswertung soll über einen mathematischen Algorithmus erfolgen. In der Beschreibung der genannten Schrift werden eine Reihe möglicher Methoden genannt, die die Grundlage zur Entwicklung eines solchen Algorithmus sein können (s. Seite 7, Zeile 12 - Seite 9, Zeile 21), ohne dass tatsächlich ein Auswertungsalgorithmus offenbart wurde. Die technische Lehre der Schrift betrifft daher nur ein Verfahren zum Suchen eines Auswertungsalgorithmus.

Ein Lösungsansatz zur lückenlosen diagnostischen Erfassung/Befundung aller Phasen der polyp-cancer-sequence wird jedoch im Stand der Technik nicht beschrieben.

Aufgabe war es daher, einen Test zur Erkennung von Darmkrebs und dessen Vorstufen zu entwickeln, der es ermöglicht, als einzelner Test alle diagnostisch erfassbaren Phasen der polyp-cancer-sequence zuverlässig und lückenlos nachzuweisen. Der Test sollte die synchrone Bestimmung von Hämoglobin und Tumor M2-PK ermöglichen.

Die Aufgabe wird gelöst durch das erfindungsgemäße Verfahren zur Diagnose/Befundung einer pathologischen Veränderung des Darms mittels eines Testkits (Kombi-Schnelltest) durch den spezifischen Nachweis von Hämoglobin und Tumor M2-PK. Mit Hilfe des erfindungsgemäßen Verfahrens ist es möglich, bereits beim Auftreten von einem der oben genannten Analyten (Hämoglobin, Tumor M2-PK) pathologische Veränderungen sehr früh sicher nachzuweisen.

Überraschenderweise hat sich gezeigt, dass sich durch die erfindungsgemäße Verwendung des Kombi-Schnelltests und zwei neuer, verbesserter Teststreifen für die Bestimmung von M2-PK und Hämoglobin, sowie die nötigen Reagenzien zur synchronen Durchführung des immunchromatographischen Nachweisverfahrens das technische Problem, die Erhöhung der Gesamtsensitivität im Vergleich zur Anwendung zwei Biomarker auf zwei getrennten oder einer Kassetten lösen lässt.

Die Erfindung betrifft deshalb im Besonderen einen Kombi-Schnelltest und eine Kombi-Schnelltestkassette und Bestandteile sowie die nötigen Reagenzien zur synchronen Durchführung eines immunchromatographischen Nachweisverfahrens. Synchron in der sprachlichen Bedeutung von gleichzeitig aber auch synchron in der technischen Bedeutung der gemeinsamen, optimalen Abstimmung der Wirkpaarungen, chromatographischen Bedingungen (insbesondere für die zwei neuen Teststreifen) zum Nachweis der beiden Biomarker M2-PK und Hämoglobin in einer Kombi-Schnelltestkassette.

Dieses war jedoch nur durch die Optimierung der Kassette und aller Bestandteile (Auswahl der Membran und Spezifikation (kapillare Flussrate usw.)), die Auswahl des sogenannten Backings, die Auswahl von Detergenzien und Lösungsvermittlern, Liquid Spreading vs. Protein-Spreading, Spezifität der ausgewählten Membran (z.B. Nitrozellulose vs. Nylon), die Auswahl des Sample Pads Selection and Specification, Conjucate Pads Selection and Specification, Absorbent Pad Selection and Specification, Ahesive Card Selection and Specification, Housing (Plastikkassette, Doppelkassette) Selecting and Specification, Manufacturing Schemes, möglich.

In-vitro-Diagnostik-Teststreifen basierend auf dem immunochromatographischen Prinzip sind seit langem Stand der Technik. Betrachtet man einen immunochromatographischen Teststrip vom Design-Stadium durch alle Produktverbesserungen zum finalen Herstellungsprozess, so werden alle Prinzipien von Biologie, Chemie, Physik und Ingenieurwissenschaften verwendet.

Folgende Patentnummern beschreiben hierzu relevante technische Lehre:

z.B. US 433734, US 4376110, US 4435504, US 4703017, US 4855240, US 4954452, US 5028535, US 5075078, US 95/16207, US 5654162, EP 0810436A1,

Testkassetten mit mehr als einem Teststreifen (Kombi-Test-Kassette) in einer Testkassette gehören zum Stand der Technik (Drogennachweise mittels Streifentest). Auch sind diese Kassetten für die Bestimmung des Hämo-/Haptoglobin Komplex und Hämoglobin bekannt. Für die synchrone Bestimmung von M2-PK und Hämoglobin sind bisher nur Einzeltestkassetten bekannt. Beide Tests allein "übersehen" viele pathologische Veränderungen. Der Grund hierfür ist unter Anderem der "schlecht" eingestellte cut-off des Teststreifens und die schlecht ausgewählten chromatographischen Bedingungen des Testsystems. Eine Herausforderung für den Hersteller eines Testsystems ist die optimale Wahl des sogenannten "cut-offs". Dieser cut-off lässt sich grundsätzlich dem Histogramm der Messwerte der Probandenpopulation entnehmen. Das technische Problem ist die Unterscheidung von "gesunden" und "kranken" Probanden. Dieses wird durch den Testkit und die Bereitstellung der beiden neuen Test-streifen mit verbessertem cut-off und der chromatographischen Bedingungen technisch gelöst. Die Bedeutung des cut-offs wird in einer Studie von van Rossum erläutert, ohne dass ein Testkit bekannt wäre, welcher die Messung von Hämoglobin und Tumor M2-PK unter Anwendung eines optimierten cut-offs zeigen würde.

Aufgabe der vorliegenden Erfindung ist es, den cut-off und die schlecht ausgewählten chromatographischen Bedingungen zu verbessern. Dieses wird erfindungsgemäß durch die Bereitstellung eines Testkits, Kombi-Schnelltestkassette, welche zwei neue Teststreifen mit "gut" eingestelltem cut-off beinhaltet, sowie die gut ausgewählten chromatographischen Bedingungen zur Ausführung des Kombi-Schnelltests technisch gelöst.

Aus den Literaturstellen WO 01/21826 A2, WO 02/50546 A2 und WO 03/069343 A2 sind verschiedene Verfahren zum Nachweis von Tumormarkern für Tumore des Gastrointestinaltrakts bekannt, bei welchen der Stuhl eines Probanden auf den Tumormarker untersucht wird. Diese Verfahren haben sich in der Praxis bewährt, weisen allerdings noch eine hohe Anzahl von falsch-negativen und/oder falsch-positiven Ergebnissen auf. Dies ist verbesserungsfähig.

Auf der anderen Seite sind verschiedene Verfahren zum Nachweis von Tumormarkern für Tumore bekannt, welche auf einem Nachweis der Tumormarker in Blut bzw. Serum basieren (siehe z. B. Anticancer Research 19:2785-2820 (1999); WO 03/065003 A2; Leman ES et al., Cancer Res. 67(12):5600-5605 (2007) und Leman ES et al., Clinical Cancer Research 14:1349-1354 (2008)). Während dies einerseits vorteilhaft ist, um eine Tumorerkrankung überhaupt zu diagnostizieren, lässt andererseits der Nachweis eines Biomarkers für verschiedenste Tumorerkrankungen keine sichere Aussage über die Art und die Lokalisation des betroffenen Organs oder Gewebes zu, da Tumormarker in den seltensten Fällen wirklich gewebespezifisch sind und damit bei einem positiven Ergebnis einer Blutanalyse auf den Tumormarker kein sicherer Rückschluss auf das betroffene Gewebe möglich ist. Es sind also zusätzlich aufwändige und komplizierte Diagnosemethoden notwendig, um die tatsächlich vorliegende Tumorart sicher festzustellen. Marker im Blut, Plasma oder Serum werden daher hauptsächlich zur Therapie- und Verlaufskontrolle von Tumoren eingesetzt.

Darüber hinaus setzt dieses Nachweisverfahren voraus, dass im Tumor gebildete Tumormarker in ausreichender Menge in die Blutbahn, bzw. in das Serum gelangen. Im frühen Stadium der Krebsentstehung, insbesondere bei Darmkrebs, gelangen noch nicht ausreichende Mengen dieser Tumormarker in die Blutbahn, so dass eine Detektion über Blut- bzw. Serumuntersuchungen nicht möglich ist.

**Synchroner Nachweis von Biomarkern**

Darüber hinaus bedarf es zum synchronen, präzisen Nachweis der beiden erfindungsgemäßen Biomarker (M2-PK und Hämoglobin) zusätzlich noch der technischen Lösung zur gemeinsamen Abstimmung der Bestimmung (des zuverlässigen Nachweises = keine Überschreitung des statistischen Konfidenzintervalls) beider Biomarker in einer Testkassette. Das heißt die gemeinsamen chromatographischen Bedingungen

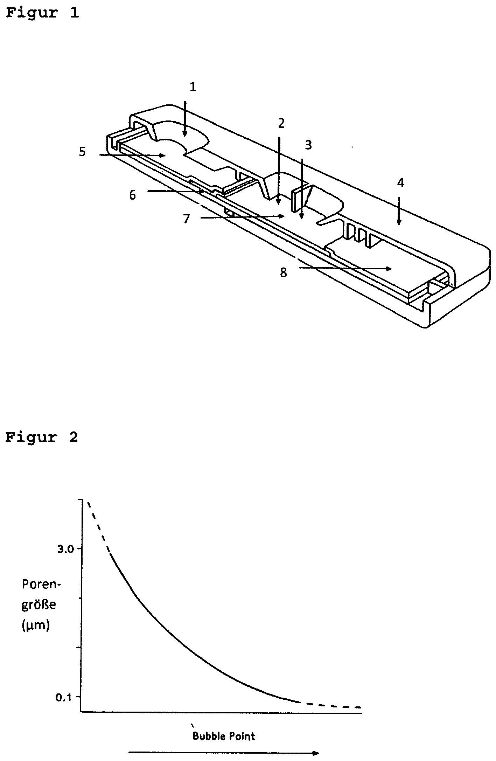

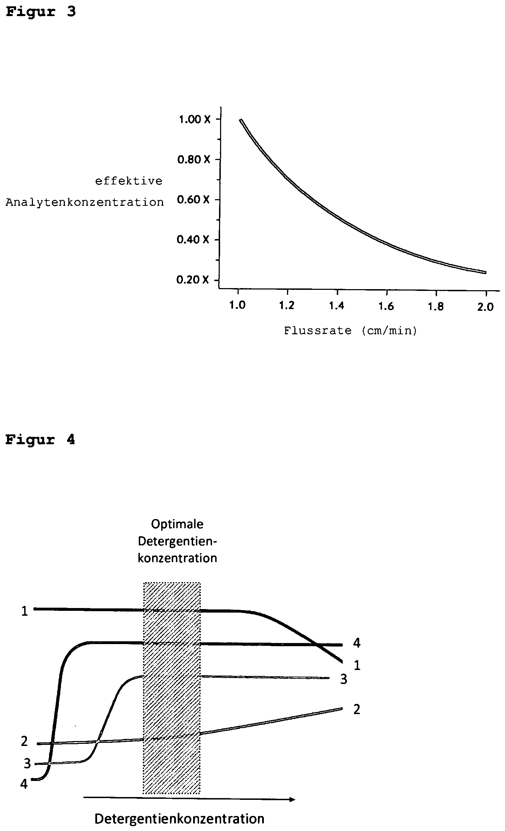

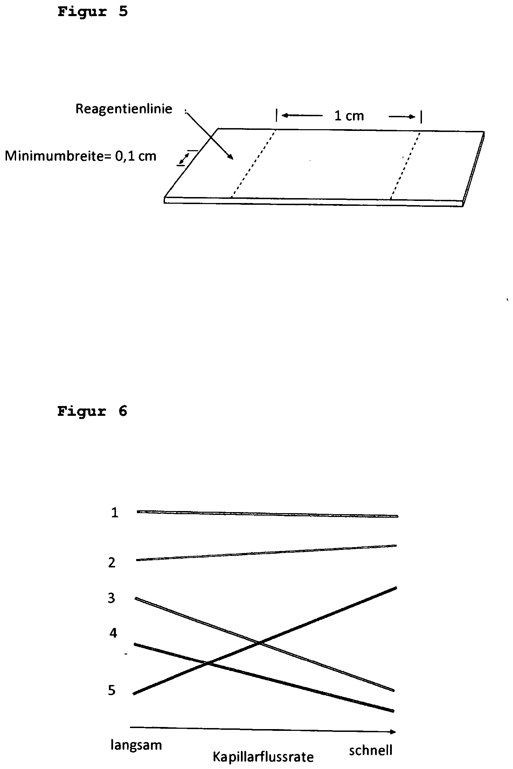

Siehe Figur 1 bis Figur 6

Dieses wird erreicht durch Auswahl der gemeinsamen, optimalen Abstimmung der chromatographischen Bedingungen zum Nachweis der beiden Biomarker M2-PK und Hämoglobin in einer Kombi-Schnelltestkassette im Sinne einer erfinderischen Parameterauswahl.

Insbesondere durch die Optimierung der Kassette und deren Bestandteile (Auswahl der Membran und Spezifikation (kapillare Flussrate usw.), die Auswahl des sogenannten Backings, die Auswahl von Detergenzien und Lösungsvermittlern, Liquid Spreading vs. Protein-Spreading, Spezifität der ausgewählten Membran (z.B. Nitrozellulose vs. Nylon), die Auswahl des Sample Pads Selection and Specification, Conjugate Pads Selection and Specification, Absorbent Pad Selection and Specification, Ahesive Card Selection and Specification, Housing Selecting and Specification, Manufacturing Schemes.

Die Herstellung dieser erfindungsgemäßen Kombi-Kassette mit zwei Testfeldern (Verwendung von Nitrozellolosemembranen) erfolgt gemäß der in-vitro-Diagnostik-IVD-Richtlinie 98/78/EG und nach ISO 13485 und dem Qualitätsmanagement QM ISO 9001.

**Technisches Problem:** schlechte klinische Sensitivität und Spezifität durch angewendete Screeningverfahren (gFOBT, iFOBT, Tumor M2-PK, Screening-Koloskopie.

**Aufgabe der Erfindung:** Verbesserung der klinischen Sensitivität (positive predictive value) und Spezifität(negative predictive value), insbesondere der Sensitivität

**Technische Lösung:** Verwendung der erfindungsgemäßen Kombi-Test-Kassette wodurch wesentliche Vorteile (Erhöhung der Gesamtsensitivität) gegenüber der Verwendung von zwei Testkassetten (eventuell von zwei unterschiedlichen Herstellern) erreicht werden. Nach dem Stand der Technik existiert im Markt ein Lateral-Flow-Test (ScheBo M2-PK Quick Test) zur gerichteten Hinführung der asymptomatischen Probanden zur Koloskopie.

**Technisches Problem:** Verbesserung der Sensitivität und Spezifität.

**Technische Lösung:** Verwendung der erfindungsgemäßen Kombi-Test-Kassette, welche die synchrone immunchemische Bestimmung der Biomarker okkultes Blut und Tumor M2-PK ermöglicht.

Die erfindungsgemäße Kombi-Test-Kassette beinhaltet zwei Lateral-Flow-Teststreifen. Beide Teststreifen entsprechen nicht dem Stand der Technik, sondern stellen jeweils erfindungsgemäß bessere Lateral-Flow-Testverfahren dar. D.h. auf der Kombi-Test-Kassette befindet sich in der bevorzugten erfindungsgemäßen Ausführungsform ein optimierter Tumor M2-PK-Test = M2-PK plus Test und ein nach dem Stand der Technik besserer iFOB-Test= iFOBplus Test.

Der synchrone (im technischen Sinne) Nachweis von den Biomarkern Tumor M2-PK und Hämoglobin mit aufeinander abgestimmten Cut-Offs in einer Kombi-Kassette (ein Testkit mit allen benötigten Reagenzien und Bestandteilen zur Testdurchführung) weist Vorteile gegenüber der zeitlich versetzten Abarbeitung (Bestimmung) von den beiden Biomarkern (Tumor M2-PK und Hämoglobin) mittels zweier Lateral-Flow-Kassetten (zwei Testkits mit zwei unterschiedlichen Zusammenstellungen von Reagenzien und Bestandteilen zur Testdurchführung) auf, da die speziellen Festphasen-/Flüssigphasenantikörper als auch sämtliche benötigten Reagenzien und Bestandteile des Kombi-Testkits aufeinander abgestimmt sind. Beispiel: würde ein Anwender den ScheBo Lateral-Flow-Test zur Bestimmung der Tumor M2-PK kaufen und einen anderen Lateral-Flow-Test zur Bestimmung des Hämoglobins von einem anderen Hersteller beziehen und zeitversetzt abarbeiten, so würde er mittels dieser zwei verschiedenen und nicht aufeinander abgestimmten Tests eine schlechtere klinische Sensitivität und Spezifität erzielen.

Aufgaben und technische Lösungen der Erfindung:

1. Senkung von Gesundheitsausgaben durch eine gezielte Zuführung zur Koloskopie.

2. Bereitstellung einer gezielten, einfachen, handhabbaren "Einstiegsdiagnostik" zur Früherkennung von Darmkrebs.

3. Verbesserung der Prognose einer individuellen Darmkrebserkrankung durch Früherkennung mittels Bereitstellung der erfindungsgemäßen Kombi-Schnelltest-Kassette zur synchronen Bestimmung der Tumor M2-PK- und iFOB-Bestimmung zur optimierten Auswahl solcher Individuen (Probanden), die einer gezielten Einstiegsbefundung durch Koloskopie bedürfen. Das technische Problem der Vorselektion zur Koloskopie lässt sich mit der erfindungsgemäßen Kombi-Schnelltest-Kassette lösen. Nur die Probanden, die bei der Messung einer der Analyten (Tumor M2-PK und/oder iFOB-Test im Testergebnis positiv befundet wurden, werden gezielt der Koloskopie zugeführt. Die erfindungsgemäße Kombi-Schnelltest-Kassette fungiert technisch gesehen als doppeltes "Filter" im Selektionsprozess.

4. Bereitstellung einer erfindungsgemäßen Lateral-Flow-Doppeltestkassette und aller dazugehörigen Reagenzien, spezieller Antikörper (anti-Tumor M2-PK (spezielles Epitop), anti-Hämoglobin (spezielles Epitop), die spezielle physiko-chemische Eigenschaften z.B. spezielle Bindungskonstante und spezielle kinetische Charakteristika besitzen. Ferner besitzen die ausgewählten Festphasen- und Flüssigphasen-Antikörper, welche gegen die beiden unterschiedlichen Analyten gerichtet sind, z.B. im Lateral-Flow-Messverfahren besondere Eigenschaften (z.B. Verträglichkeit mit Glasfaser, Nitrozellulose-Membran, gute Kopplungseigenschaften an z.B. Gold-Kolloid).

5. Verwendung von besonderen Antikörpern im Kombi-Schnelltest, die sowohl spezifisch in ihrer Bindungseigenschaft für Tumor M2-PK oder Hämoglobin sind und in immunchromatographischen Verfahren verwendbar sind (z.B. "membrantauglich", "detergenztauglich" sind).

6. Senkung der Letalität von Darmkrebs durch die erfindungsgemäße Bereitstellung eines Kombi-Schnelltests zur gezielten Hinführung zur invasiven Methode - Koloskopie - und damit zielgerichteter Vorbereitung (gemäß "diagnostischem" - Maßnahmen- Entscheidungsbaum/Entscheidungsmatrix) zu einem therapeutischen Verfahren (Chirurgie, Chemotherapie).

7. Kosten-/Nutzenoptimierung der Screeningmaßnahmen bezüglich der Erkennung von Darmkrebs und dessen Vorstufen.

Aus der Literaturstelle WO 01/21826 ist bekannt, dass man die Tumor M2-PK auf einem Teststreifen mittels eines chromatographischen Verfahrens nachweisen kann. So kann beispielsweise bei diesem Test ein Teststreifen (Nitrozellulose) verwendet werden, auf dem in unterschiedlichen Zonen des Teststreifens die benötigten Antikörper entweder in löslicher Form oder festphasenfixiert angeordnet sind. Die Probe (z.B. Stuhlextrakt, eine Mischung aus vielen Stoffen, aber auch dem nachzuweisenden Analyten in Detergenslösung) bzw. der Flüssigkeitsanteil der Probe oder ein Extrakt können den Teststreifen durchwandern und an der Detektionsstelle ein Signal liefern, wenn z.B. Tumor M2-PK und/oder Hämoglobin in der Probe vorhanden ist. Die erfindungsgemäße Verwendung von hochspezifischen Fangantikörpern stellt die Bindung der Zielproteine (z.B. Tumor M2-PK) aus dem Stuhlprobenextrakt sicher. Durch die Verwendung von Goldkolloiden an denen sich ein zweiter hochspezifischer Antikörper befindet, bildet sich bei Anwesenheit von z.B. Tumor M2-PK ein Immunkomplex. Dieser Analyt-Nachweis-Komplex ist mit bloßem Auge auf dem Teststreifen als Linie sichtbar. Die genaue Anordnung der einzelnen Komponenten auf einem Teststreifen ist abhängig vom angewandten immunologischen Verfahren und dem Fachmann bekannt. Eine besondere Herausforderung ist die Verwendung der optimalen Antikörper. Bei dem erfindungsgemäßen Kombi-Schnelltest wurden spezielle Antikörper, die speziell die Tumor M2-PK binden, verwendet.

Der Tumor M2-PK-spezifische Antikörper bindet (auch stereospezifisch die dimere Form der M2-PK) bevorzugt an eines der folgenden Epitope oder Fragmente von diesem, die mindestens eine Länge von 4 Aminosäuren haben:

| | |
|---|---|
| LAPITSDP | EAEAAIYH |
| VEASFKCC | SGAIIVLT |
| CSGAIIVLT | LQLFEE |
| TEATAVGA | QLFEELRR |
| LRRLAPITSDPTEATA | VEASFKC |
| KCCSGAIIV | KSGRSAHG |

Das Auftreten eines dieser Epitope oder Fragmente von diesen, welche mindestens eine Länge von 4 Aminosäuren haben, oder Kombinationen von diesen in einem Stuhlprobenextrakt, ist ein Hinweis auf krankhafte Veränderungen (Darmkrebs oder dessen Vorstufen) im Gastroinstestinaltrakt.

Die sog. Blut-im-Stuhl Tests (gFOBT) zeigen eine ungenügende Sensitivität von lediglich 25 % für die Erkennung von Darmkrebs. Der Tumor M2-PK Test zeigt gegenüber diesen eine höhere Sensitivität von 80 %. Mit dem Tumor M2-PK Test können Polypen größer 1 cm mit einer Sensitivität von 60 % (FOBT: 20 %), Polypen kleiner 1 cm mit einer Sensitivität von 20 % (FOBT: 0 %) nachgewiesen werden (Alle Werte bei einer Spezifität von 95 %).

Generell zeigen immunchromatographische Blut-im-Stuhl Tests eine höhere Sensitivität für Darmkrebs und dessen Vorstufen als die normalerweise benutzten gFOBTs, daher sind die gFOBTs den iFOBTs in der Aussagekraft unterlegen.

Die positiven Raten sowie die Spezifität und die Sensitivität unter diesen Tests variieren stark. Die beobachteten Muster legen nahe, dass die stark unterschiedlichen Positivraten auf verschiedenen cut-off Levels, unterschiedlichen Grenzbereichen und Grenzwerten der Tests der verschiedenen Hersteller aus verschiedenen Ländern beruhen. Auch die Herstellungsqualität der Tests unterschiedlicher Hersteller ist unterschiedlich.

Testkits unterschiedlicher Hersteller adressieren unterschiedliche cut-offs (=Toleranzgrenze sie bezeichnet einen Toleranzwert). Der Toleranzwert legt fest, ab wann ein Testergebnis positiv bzw. negativ zu bewerten ist. Der cut-off ist von dem Begriff der Nachweisgrenze zu unterscheiden.

Lit: Inter-test agreement and quantitative crossvalidation of immunochromatographical fecal occult blood tests, Hermann Brenner et al.; Int. J. Cancer (2010)

Eine Folge davon ist eine zu beobachtende starke Variation in den Testcharakteristiken einzelner Tests (technisches Problem). Hieraus wird in der wissenschaftlichen Literatur gefordert, bessere Testkits zur Verfügung zu stellen und dabei optimierte cut-offs zu finden und anzuwenden (H. Brenner, S. Tao, European Journal of Cancer (2013).

Weitere Studien untermauern die hohe Varianz von Testkits, die auf dem Markt erhältlich sind (Brenner et al, Int. J. Cancer, 2010). Daraus resultiert ein hohen Bedürfnis der Fachwelt für ein Testkit, welches in der Lage ist, Tumor M2-PK und Hämoglobin in Stuhlproben anzuzeigen unter Verwendung eines cut-offs, der eine möglichst hohe diagnostische Effizienz gewährleistet.

Gelöst wird dieses technische Problem durch die Bereitstellung des Testkits enthaltend die Kombikassette, die zwei, nach dem Stand der Technik verbesserte Teststreifen enthält.
Cut-Off levels, Grenzbereiche und Grenzwerte für beide Biomarker wurden durch aufwändige firmeninterne Messungen gefunden und festgelegt. Die Festlegung erfolgte durch deskriptive statistische Analyse und Interpretation der Biomarker-Werte- Histogramme (Box-Plot- Analysen und Kruskal-Wallis- Berechnungen). Die Herstellung beider Teststreifen sowie der Anpassung der chromatographischen Bedingungen für die synchrone Bestimmung beider Biomarker zur Erreichung besserer klinische Sensitivitäten und Spezifitäten, stellt eine große Herausforderung dar. Dieses wurde technisch durch die Anpassung sämtlicher chemischer und physikalischer Bedingungen (siehe Ansprüche und Beispiele) gelöst. So wurden alle Bedingungen zur Bestimmung der beiden Biomarker so gewählt, das möglichst wenig "falsch positive" bzw. "falsch negative" Resultate bei der Durchführung des Kombitests auftreten, um auf diese Weise die diagnostische Effizienz, welche das Verhältnis aller richtig positiven und richtig negativen Testergebnisse zur Gesamtheit aller Ergebnisse angibt, deutlich erhöht.
Zur Erreichung dieses Ziels wurde eine technische Lösung gesucht und gefunden. Für die technische Lösung ergab sich der im technischen Sinne synchrone Nachweis der beiden Biomarker unter abgestimmten chromatographischen und abgestimmten materiellen Bedingungen D.h. richtige Wahl der Membrane, Laufbedingungen, usw. (siehe Beispiele und Ansprüche).

Tumormarker sind Genprodukte, die in Tumorgewebe differenziell gegenüber normalem Vergleichsgewebe exprimiert werden, i.e. ein Tumormarker wird in Tumorgewebe gegenüber normalem Gewebe entweder über- oder unterexprimiert. Für die Praxis sind Tumormarker wichtiger, welche überexprimiert werden, da dann ein positives Analysenergebnis (Vorliegen des Biomarkers) einer Probe indizielle für das Vorliegen des Biomarker-spezifischen Tumors ist. Tumormarker liegen im Zytoplasma der Gewebezellen vor, können jedoch auch in Körperflüssigkeiten, wie Blut, Urin, Schweiss, Sperma, Speichel usw. aber auch im Stuhl vorkommen.

Ein Tumormarker sollte folgende Eigenschaften haben: i) hohe Spezifität, d. h. bei benignen Erkrankungen und gesunden Personen nicht nachweisbar, ii) hohe Sensitivität, d. h. er kann in einem hohen Prozentsatz der Tumorpatienten nachgewiesen werden, iii) Organspezifität, iv) gute Korrelation mit den Tumorstadien bzw. der Tumormasse, v) Beziehung zur Prognose und vi) verlässliche Vorhersagewerte. Die Kriterien der 100 % igen Spezifität und der 100 % igen Sensitivität sowie die anderen aufgeführten Kriterien werden bis heute von keinem der bekannten Tumormarker erfüllt.

U.a. für Tumore des Gastrointestinaltraktes spezifische Tumormarker umfassen CCSA-2, CCSA-3, CCSA-4, CC2, CC3, CC4, CC5, CC6a, CC6b, L1, L2, N1, N2, N3, N4, N5, und N6 (siehe z. B. WO 03/065003 A2; Leman ES et al., Cancer Res. 67(12):5600-5605 (2007) und Leman ES et al., Clinical Cancer Research 14:1349-1354 (2008)). Diese Tumormarker werden im Stand der Technik jedoch nur im Rahmen von Blut- bzw.- Plasmatests verwendet, was zu den vorstehend genannten Nachteilen führt.

Es ist wünschenswert, möglichst frühzeitig ein tumoröses Geschehen im Gastrointestinaltrakt, insbesondere in einer Wachstumsphase, in der der Tumor noch keinen Kontakt mit dem Gefäßsystem des Körpers aufgenommen hat (beispielsweise bereits in der "Polyp-cancer-sequence", i.e. zeitlich vor der Infiltration der Submukosa) nachweisen zu können. Beim Verdacht auf ein neoplastisches Geschehen im Gastrointestinaltrakt, besonders im Hinblick auf die sogenannte Adenom-Carcinom-Sequenz bei Polypen, wird nach dem Stand der Technik mittels verschiedener Bestimmungsmethoden versucht, okkultes Blut im Stuhl nachzuweisen. Hierzu werden nicht-immunologische Tests (z. B. Pseudoperoxidase-Aktivität, Porphyrinnachweis) und immunologische Tests verwendet (Favenne L. et al. (1992) Ann. Biol. Clin. 50 : 311-313).

Beide Testprinzipien sind jedoch nicht sehr spezifisch. Zudem kann der nicht immunologische Test basierend auf einer Redox-Reaktion (Testprinzip) durch eine Vielzahl von Einflussgrößen gestört werden können (falsch positiv/falsch negativ, z. B. durch Nichteinhalten dringend notwendiger Diätvorschriften von Seiten des Patienten und von einer Reihe von Arzneimitteln sowie durch überhöhte Vitamin-C-Gabe (z. B. in Gemüse, Fruchtsäften etc.), Thomas L., Labor und Diagnose, 5. Auflage, 1998).

Ein positiver Test auf okkultes Blut im Stuhl muss so lange abgeklärt werden, bis die Blutungsquelle lokalisiert ist bzw. die Ursache der Blutung gefunden wurde. Der klinische Befund rechtfertigt in jedem Fall eine zügig durchzuführende weitergehende Diagnostik ("Ausschlussdiagnostik"), z. B. durch Endoskopie, Sonographie, Röntgen.

**Technisches Problem der Erfindung**

Der nach dem Stand der Technik existierende M2-PK Lateral Flow Test (Teststreifen) übersieht pathologische Veränderungen.

Der erfindungsgemäße **M2-PK plus Teststreifen** übersieht auch pathologische Veränderungen.

Der nach dem Stand der Technik existierende iFOBT übersieht pathologische Veränderungen.

Der erfindungsgemäße **iFOBT plus Teststreifen** übersieht pathologische Veränderungen. Bringt man beide verbesserte Tests M2-PK plus und iFOBT plus als Teststreifen erfindungsgemäß in eine Kombikassette erzielt man gemäß Aufgabe der Erfindung eine höhere Erkennungsrate für pathologische Veränderungen im Darm. Technische Problemlösungen, insbesondere die Auswahl von Wirkpaarungen (siehe Beispiele und Liste der erfindungsgemäßen Komponenten und aktiven Bestandteile, die richtige Wahl der Antikörper und die richtige Wahl der cut-offs für beide Parameter, der analytischen Empfindlichkeit, kapillare Flussraten usw.) zum synchronen Nachweis der beiden Biomarker sind Gegenstand dieser Erfindung erfinderische Parameterauswahl.

Der Erfindung liegt daher das technische Problem zu Grunde, ein einfach aufgebautes Testkit (Kombi-Schnelltest) anzugeben, welches als Verfahren zum Nachweis von Markern, Biomarkern, insbesondere Enzymbiomarkern im menschlichen oder tierischen Stuhl dient, wobei das Verfahren zum Nachweis eines malignen Geschehens im Gastrointestinaltrakt und der Bauchhöhle (Speiseröhre, Magen, Dünndarm, Gallenweg, Bauchspeicheldrüse und Dickdarm), insbesondere von Tumoren und Tumorvorstufen des Darmes (Polypen, Adenome) geeignet ist. Das erfindungsgemäße Kombi-Testkit (Kombi-Schnelltest) soll dabei den Nachweis von pathologischen Veränderungen auf einfache Weise möglich machen, so dass der Test von technischen Angestellten durchgeführt werden kann.

Der Erfindung liegt insbesondere das technische Problem zu Grunde den weiterhin steigenden Bedarf an spezifischen, leicht durchzuführenden Verfahren zu befriedigen, so dass ein pathologisches Geschehen, insbesondere ein neoplastisches Geschehen besonders frühzeitig und zweifelsfrei nachzuweisen ist, insbesondere im Hinblick auf die Problematik der sogenannten Adenom-Carcinom-Sequenz bei Polypen.

**Grundzüge der Erfindung und bevorzugte Ausführungsformen**

Zur Lösung dieses technischen Problems lehrt die Erfindung einen Testkit (Kombi-Schnelltest) zum Nachweis von Biomarkern im menschlichen Stuhl, bestehend aus einem ersten Proberöhrchen, enthaltend eine erste Stuhlprobeentnahmevorrichtung zur Aufnahme der benötigten Stuhlmenge, eine erste Pufferlösung, enthaltend 10-70 mM Hepespuffer mit pH-Wert 7,6-8,2, einem zweiten Proberöhrchen, enthaltend eine zweite Stuhlprobeentnahmevorrichtung zur Aufnahme der benötigten Stuhlmenge, eine zweite Pufferlösung, enthaltend 10-70 mM Acetatpuffer mit pH-Wert 5-6, einer Testkassette enthaltend ein Lateral-Flow Testsystem mit einer drei bis sechs Monate gelagerten Nitrozellulosemembran als stationärer Phase, die Nitrozellulosemembran wiederum enthaltend monoklonaler Tumor M2-PK Antikörper der Maus, nämlich Klon PATAM3AT, IgG1 und Gold-gekoppelter monoklonaler MausAntikörper, nämlich Klon 1 E3, IgG1, und monoklonaler Hämoglobin-Antikörper der Maus, nämlich Klon M1202100, IgG1, und Gold-gekoppelter monoklonaler Mausantikörper, nämlich Klon HB11-2312, eine erste Öffnung zum Auftragen einer Stuhlprobe aus dem ersten Proberöhrchen" eine zweite Öffnung zum Auftragen einer Stuhlprobe aus dem zweiten Proberöhrchen, wobei das Analyseergebnis der ersten Stuhlprobe positiv ist, wenn der Gehalt an Tumor M2-PK größer ist als $4 \pm 1$ Units/ml Stuhlextrakt und das Analyseergebnis der zweiten Stuhlprobe positiv ist, wenn der Gehalt an Hämoglobin 24$\mu$g Hämoglobin pro Gramm Stuhl übersteigt.

Der Testkit wird verwendet zur in-vitro Diagnostik einer pathologischen Veränderung des Darms.

Das erfindungsgemäße Verfahren zur in-vitro Diagnostik eines malignen Geschehen im Gastrointestinaltrakt unter Verwendung des Testkits ist gekennzeichnet durch Entnahme von zwei Stuhlproben mittels zweier Entnahmevorrichtungen zur Aufnahme der jeweils benötigten Stuhlmenge, Auflösung der ersten Stuhlprobe in der ersten Pufferlösung, Auflösung der zweiten Stuhlprobe in der zweiten Pufferlösung, Auftragung der ersten und der zweiten stuhlhaltigen Pufferlösung auf die jeweils zugeordnete Öffnung, Abwarten der vorgeschriebenen Wartezeit, Ablesen der Ergebnisse in den jeweiligen Ableseöffnungen. Der Testkit kann verwendet werden zum Nachweis eines pathologischen Geschehens im Gastrointestinaltrakt, wobei eine Probe menschlichen Stuhls, gelöst in einem speziellen Extraktionspuffer, auf einen Träger aufgebracht wird und darauf sowohl Tumor M2-PK, als auch Hämoglobin auf einem Träger nachgewiesen werden. Die Ablesung erfolgt hierbei auf einfache Weise visuell mit dem bloßen Auge (ohne Zuhilfenahme eines Gerätes).

In einer bevorzugten Ausführungsform der Erfindung wird Hämoglobin in Form des Hämoglobin-Haptoglobin-Komplexes (Hb-Hp) bestimmt. Es wurde festgestellt, dass die Messung der oben genannten Biomarker besonders zum Nachweis früher maligner Veränderungen, insbesondere Neoplasmen geeignet ist. Durch die Messung dieser Biomarkerkombination Tumor M2-PK plus Hämoglobin; Tumor M2-PK plus Hämoglobin-Haptoglobin-Komplex (Hb-Hp) tritt eine deutliche Verbesserung der Aussagegenauigkeit (Befundung) ein, insbesondere eine signifikante Absenkung der falsch-negativen, aber auch der falsch-positiven Befunde.

Dies ist insbesondere überraschend, weil umfangreiche Untersuchungen bzgl. anderer spezifischer Analyten (Proteine) im Stuhl keine Hinweise auf ihre Aufwendung als Biomarker, insbesondere als Tumormarker zuließen. Sowohl die Struktur, als auch die physikochemischen Eigenschaften der genannten Tumormarker werden offensichtlich infolge der erheblichen proteolytischen Aktivität und den extremen physiologischen Gegebenheiten (z. B. pH-Wert, sauer im Magen, alkalisch im Darm) des Gastrointestinaltraktes nicht nachhaltig beeinträchtigt. Dies gilt auch für den Nachweis der Biomarker mittels immunologischer Methoden. Es wurde daher festgestellt und gezeigt, dass sich trotz der oben genannten Proteindenaturierung und Proteinverdauung im Gastrointestinaltrakt ein spezifischer Nachweis mehrerer Enzymbiomarker im Stuhl von Tumorpatienten durchführen lässt.

Die für die Bestimmung der Enzymbiomarker notwendigen Systeme sind nicht ohne weiteres kommerziell verfügbar. Zur Entwicklung des erfindungsgemäßen Kombi-Schnelltest mussten sowohl die Nachweisgrenzen, als auch die Zusammensetzung der Reagenzien (erfindungsgemäß: spezielle Antikörper für die flüssige und für die feste Phase für beide Analyten optimiert für die synchrone Messung der Analyten) und Pufferlösungen, Detergentien usw., aufwändigst angepasst werden.

Bei einer M2-PK und/oder Hämoglobin positiven Stuhlprobe wird damit unmittelbar ein Hinweis auf ein pathologisches, möglicherweise neoplastisches, insbesondere malignes, Geschehen im Gastrointestinaltrakt, also insbesondere im Darm , der Speiseröhre, im Magen,, in den Gallenwegen, in der Bauchspeicheldrüse oder/und im Dickdarm, angezeigt und somit eine Lokalisation des Geschehens im Gastrointestinaltrakt ermöglicht.

Es wird darauf hingewiesen, dass eine Stuhlprobe nicht als eine "Körperflüssigkeit"-Probe betrachtet werden kann, da sich der gesamte Gastrointestinaltrakt biologisch gesehen außerhalb des Körpers befindet.

Mit dem erfindungsgemäßen Verfahren ist es nun möglich, in einem nicht-invasiven Verfahren auf einfache Weise durch Bestimmung mehrerer der besagten Tumormarker-Proteine ein malignes Tumorgeschehen im Stuhl nachzuweisen und gleichzeitig bei positiver Testführung eindeutig festlegen zu können, dass ein Tumor im Gastrointestinaltrakt und nicht an anderer Stelle im Körper vorliegt.

Mit diesem Verfahren gelingt nicht nur die Generierung eines "ersten Verdachts" "Primärdiagnose", Diagnose, sondern es kann auch eine Verlaufskontrolle vorgenommen werden. Wegen der individuellen Proteinverteilung ist eine individuelle Diagnostik möglich.

Überraschenderweise ist es mit dem erfindungsgemäßen Kombi-Schnelltest möglich, die besagten Enzymbiomarker als freie Proteine im Stuhl nachzuweisen. Dies ist auch deswegen vorteilhaft, weil die Testdurchführung durch Assistenzpersonal, beispielsweise eine medizinisch-technische Angestellte (MTA), erfolgen kann. Dadurch, dass die beiden Analyten (Tumor M2-PK und Hämoglobin) synchron bestimmt werden, werden insbesondere Ablesefehler (beispielsweise der MTA) vermieden, denn zur Testdurchführung werden die beiden Probetropfen eines Patienten nacheinander (naturgemäß mit wenigen Sekunden Verzögerung) aufgetragen und nach Ablauf einer gewissen Zeitspanne von wenigen Minuten, werden beide Ergebnisse abgelesen. Durch diese Vorgehensweise wird nicht nur eine höhere Bequemlichkeit der Testdurchführung, sondern auch eine Verringerung von Fehlerquellen (im Vergleich zu herkömmlichen Tests) erreicht.

Für den erfindungsgemäßen Test ist keine Isolierung von Zellen aus dem Stuhl und die Analyse der in den Zellen enthaltenen Proteine erforderlich. Denn es wurde herausgefunden, dass feste Tumoren die genannten Enzymbiomarker als lösliche Proteine in das Lumen des Gastrointestinaltraktes abgeben und diese Biomarker sich nicht in abgeblätterten gastrointestinalen Epithelzellen befinden. Die besagten Enzymbiomarker werden von Tumorzellen freigesetzt, durchlaufen den Gastrointestinaltrakt als Protein und können schließlich als Protein im Stuhl nachgewiesen werden. Diese Möglichkeit, nämlich dass diese Proteine nach z. B. der Magen- bzw. Darmpassage im Stuhl nachweisbar bleiben, war nicht zu erwarten, da normalerweise eine weitgehende Zersetzung der betreffenden Proteine im Zuge des normalen Verdauungsvorganges zu erwarten gewesen wäre.

Im Rahmen der Erfindung wurde festgestellt, dass besagte Enzymbiomarker quantitativ sogar auch bei intensiv homogenisierten, länger gelagerten Stuhlproben (zum Beispiel beim Probenversand) nachweisbar bleiben. Auch bei starker Verdünnung des Stuhls wird eine deutliche Reaktion erhalten. Zudem kann der Nachweis auch ohne vorherige Extraktion in der Stuhlprobe selektiv erfolgen. Vorzugsweise ist jedoch ein Extraktionsverfahren beispielsweise unter Verwendung eines speziellen Detergens (z.B. CHAPS) zu verwenden (das Extraktionsverfahren wird näher in Beispiel 2 beschrieben).

Beim erfindungsgemäßen Verfahren wird vorzugsweise das maligne Geschehen im Gastrointestinaltrakt eines Menschen bestimmt. Weiterhin werden die Tumormarker immunochemisch nachgewiesen mittels monoklonaler Antikörper, welche nicht mit anderen Bestandteilen des Stuhls, insbesondere nicht mit anderen Stuhlproteinen kreuzreagieren.

Fragmente im Sinne der Erfindung sind Proteine oder Polypeptide mit einer Aminosäuresequenz, welche identisch mit einer Teilsequenz eines der besagten Tumormarker sind. Die Länge eines Fragments kann von 5 aa (aa = Aminosäuren) bis zu n-1 aa (n = Länge des Tumormarkers, aus welchem das Fragment herrührt) betragen. Typischerweise wird die Länge mehr als 10 aa betragen.

Erfindungsgemäß einsetzbare Antikörper können gemäß dem Stand der Technik bekannten Verfahren hergestellt werden. Dem Fachmann sind Verfahren zur Herstellung von spezifischen monoklonalen Antikörpern gut bekannt. Beispielsweise wird hierzu das Antigen, im vorliegenden Fall also ein Enzymbiomarker, zur Erzeugung von Antikörpern verwendet. Prinzipiell kann hierzu das Verfahren, welches erstmals von Koehler und Milstein beschrieben wurde, angewandt werden, wobei dem Fachmann auch Abwandlungen und Weiterentwicklungen dieser Verfahren bekannt sind. Mit dieser Herstellung können spezifische Antikörper erhalten werden, wobei die Spezifität durch Selektion festgestellt werden kann. Die Selektion erfolgt auf spezifische Antikörper, welche an einen Enzymbiomarker, jedoch nicht an andere Stuhlproteine binden. Wie beschrieben, erfolgt der Nachweis nach dem Prinzip des Immunoassays. Ein Immunoassay wird in der Weise durchgeführt, dass man a) die Probe mit mindestens zwei verschiedenen Rezeptoren in Kontakt bringt, von denen der erste Rezeptor R1 in fester Phase vorliegt und mit dem Tumormarker bindefähig ist und der zweite Rezeptor R2 in flüssiger Phase vorliegt und ebenfalls mit dem gleichen Enzymbiomarker bindefähig ist, wobei der Rezeptor R2 eine Markierung trägt oder die Bindung an ein detektierbares Molekül vermittelt, b) die feste von der flüssigen Phase trennt, und c) die Markierung oder das detektierbare Molekül in einer der Phasen, bevorzugt in der festen Phase bestimmt und entsprechend die Menge an in der Probe vorhandenen Enzymbiomarkern quantifiziert, wobei man als mindestens einen der Rezeptoren R1 oder R2 einen Antikörper verwendet, der spezifisch mit Tumor M2-PK bindefähig ist und insbesondere an kein anderes Stuhlprotein bindet. Es wird sowohl als Rezeptor R1 als auch als Rezeptor R2 ein Antikörper gegen Tumor M2-PK eingesetzt. Unter Tumor M2-PK wird erfindungsgemäß die dimere Form dieses Enzymbiomarkers verstanden, welche sich von der tetrameren Form unterscheidet.

Die Bestimmung des Hämoglobins erfolgt gemäß dem oben für Tumor M2-PK dargestellten Testprinzip.

Neben der Durchführung des Verfahrens in Form eines Immunoassays und insbesondere eines ELISA können auch andere Nachweistechnologien eingesetzt werden, z. B. die Schwingquarz-Technologie, Mikrowaage-Technologie, Lateral-Flow-Technologie, Kandelaber-Technologie, TRACE-Technologie oder Elektrochemilumineszenz-Technologie, aber auch die Agglutination mit Mikro- oder Nanopartikeln (Messung durch Nephelometrie) sowie Multiplex-Technologien an der Flüssig- oder Festphase oder Array-Technologien, wie beispielsweise mittels Proteinchips. Diese Technologien sind dem Fachmann gut vertraut und brauchen daher hier nicht näher erläutert zu werden. Erfindungsgemäß wird die Lateral-Flow-Technologie eingesetzt. Gegenstand der vorliegenden Erfindung ist daher ein Kombi-Schnelltest zum Nachweis und/oder zur Verdachtsdiagnose eines malignen Tumorgeschehens im Gastrointestinaltrakt in Menschen, welcher insbesondere zur Durchführung des oben beschriebenen Verfahrens vorgesehen ist, mittels Bestimmung einer Enzymbiomarkerkombination, wobei der Kombi-Schnelltest Synchron Tumor M2-PK und Hämoglobin oder Hämoglobin/Haptoglobin bestimmt. Es enthält der Kombi-Schnelltest einen Antikörper für Tumor M2-PK, der mit keinem anderen Stuhleiweiß kreuzreagiert und einen Antikörper der spezifisch Hämoglobin/Haptoglobin erkennt und mit keinem anderen Stuhleiweiß kreuzreagiert. Das Testkit kann gegebenenfalls weitere für die Durchführung eines Immunoassays oder für die Durchführung des jeweils vorgesehenen Testverfahrens notwendige Reagenzien enthalten. Es enthält das Testkit einen an eine Festphase gebundenen, für mindestens einen der genannten Enzymbiomarker spezifischen Antikörper. Beschrieben sind sind Antikörper, insbesondere monoklonale Antikörper, welche spezifisch einen der besagten Enzymbiomarker binden und vorzugsweise mit keinem anderen Stuhlprotein kreuzreagieren. Die Antikörper können beispielsweise nach der Methode von Koehler und Milstein (Nature 256,495-497 (1995)) erzeugt werden.

Weiterhin beschrieben sind Aptamere oder Spiegelmere und deren Verwendung an Stelle der vorstehend beschriebenen Antikörper (es gelten alle Antikörperbezogenen Ausführungen analog), welche spezifisch an einen der besagten Enzymbiomarker binden und optional mit keinem anderen Stuhlprotein kreuzreagieren. Aptamere sind Oligonukleotidsequenzen, welche spezifische Bindeeigenschaften aufweisen. Solche Aptamere können beispielsweise nach den in US 5,270, 163 oder in Sumedha, Clin. Chem. 45 (1999), 1628-1650 beschriebenen Methoden hergestellt bzw. identifiziert werden. Spiegelmere sind Aptamere, welche aus L-Oligonukleotiden gebildet sind.

Die Auswertung der erfindungsgemäßen Tests erfolgt unter Nutzung der automatischen Klassifikation, Clusteranalyse, Mustererkennung. Insbesondere werden Verfahren der "maximum-likelihood-Methode" bzw. der Clusterzugehörigkeit über Wahrscheinlichkeitsverteilungen angewandt.

Für das Laborpersonal und den behandelnden Arzt wird das Ergebnis bevorzugt in Form in einer mehrfarbigen Darstellung präsentiert (Risiko-Ampel), Entscheidungsmatrix und weiteren Algorithmen zur Entscheidungsfindung.

Beispielsweise können die gemessenen Parameter (M2-PK und Hämoglobin) folgendermaßen zugeordnet werden:

|  | M2-PK | Hb | Bedeutung |
|---|---|---|---|
| Stufe 1 (grün) | - | - | Darmkrebs kann mit >97%iger Sicherheit ausgeschlossen werden |
| Stufe 2 (gelb) | - | + | Weitere Abklärung geboten (z.B. Koloskopie) |
| Stufe 3 (orange) | + | - | Weitere Abklärung geboten (z.B. Koloskopie) |
| Stufe 4 (rot) | + | + | 90% ige Wahrscheinlichkeit für fortgeschrittene Neoplasien |

In jedem Fall enthält ein erfindungsgemäßes Testkit eine Probenentnahmevorrichtung für Stuhl. Hierfür kommen alle fachüblichen Vorrichtungen in Frage, beispielsweise die in der DE 102 05 709 A1beschriebenen Vorrichtungen. Eine besondere Herausforderung zur Erreichung des optimalen Funktionsprinzips war die Auswahl der optimalen Wirkpaarungen. Diese Wirkpaarungen werden in den folgenden Beispielen, Listen und Patentansprüchen und im besonderen in den Figuren 1-6 angegeben.

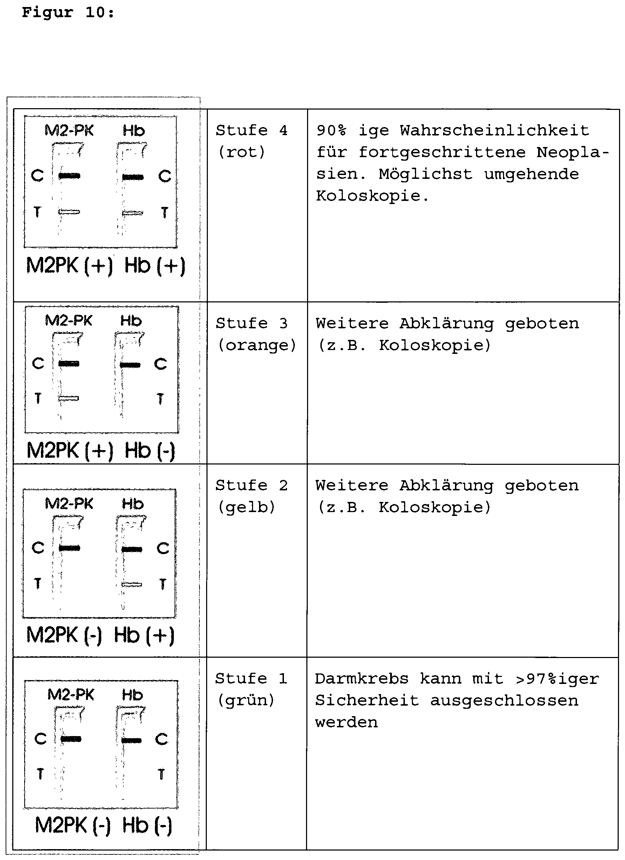

Z.B Figur 2, 3, 4 und 6 stellen unterschiedliche Funktionen dar. Eine besondere Herausforderung dieser Erfindung war im besonderen unter den zahlreichenden verschiedenen Funktionen (Kombinationsmöglichkeiten) die optimalen Wirkpaarungen zur Erreichung des optimalen Funktionsprinzip auszuwählen (Auswahl von Parametern). Beipsielsweise findet die Auswertung automatisch statt. Beispielsweise kann das erfindungsgemäße Testkit fotografiert und das Testergebnis über ein Auswerteprogramm der entsprechenden Risikostufe zugeordnet werden. Beschrieben sind derartige Vorrichtungen zum Ablichten des Testkits sowie die Auswerteprogramme zur Darstellung des Testergebnisses, vorzugsweise in Form einer mehrfarbigen Darstellung (Risiko-Ampel). Beispielhaft ist die Zuordnung in Figur 10 dargestellt.

## Beispiele

### Beispiel 1 : Testkit

Das Testkit auf Basis eines Immunossays besteht aus zwei Probeentnahme- und vorbereitungsvorrichtungen sowie einer Testkassette.

Beide Probeentnahmevorrichtungen enthalten ein Stäbchen, welches die benötigte Stuhlmenge (4-30 mg , bevorzugt 25 mg) aufzunehmen vermag. Die Probeentnahmevorrichtungen enthalten weiterhin jeweils ein Röhrchen zur Probenaufnahme, welche mit Pufferlösung gefüllt sind.

Die wässrige Pufferlösung für den Tumor M2-PK-Test enthält folgende Bestandteile:

Puffer 1 = 10-70 mM Phosphatpuffer (pH 6,7-7,6) oder Puffer 2 = 10-70 mM Hepespuffer (pH 7,6-8,2) oder Puffer 3 = 10-70 mM Triäthanolamin (pH 7,3-7,7) bevorzugte Mischungen hiervon jeweils Volume 1:1:1

Detergenz 1 = CHAPS (3-[(3-Cholamidopropyl)dimethylammonio]-1-propansulfonat, 10 mM-50 mM (Sigma), Detergenz 2 = Sodium Dodecyl Sulfate (SDS) 0,01% - 0,1 %), z.B. von Biochrom, Detergenz 3 = Lauryldimethylamine Oxide (10 mM-50mM), z.B. von Biochrom oder Mischungen hiervon,

Die wässrige Pufferlösung für den Hämoglobin-Test enthält folgende Bestandteile:

Puffer 1 = 10-70 mM Phosphatpuffer (pH 6,7-7,6) oder Puffer 2 = 10-70 mM Hepespuffer (pH 7,6-8,2) oder Puffer 3 = 10-70 mM Triäthanolamin (pH 7,3-7,7) oder Mischungen hiervon.

### Verwendete Antikörper

Die zur Messung benötigten 4 Antikörper (jeweils 1 in der "Flüssigphase" und 1 Antikörper in der "festen Phase") befindliche spezifisch bindefähige Antikörper können jeweils polyklonal bevorzugt jedoch monoklonal sein.

Die polyklonalen, als auch die monoklonalen Antikörper sind erhältlich nach dem Stand der Technik durch die klassischen Methoden der Immunisierung von Tieren mit dem jeweiligen Antigen oder bevorzugt durch die Verwendung der HybridomaMethode nach Köhler und Mielstein.

Polyklonale, als auch monoklonale Antikörper, die die menschliche Pyruvatkinase auch ihre Isoenzyme der Pyruvatkinase binden, gehören zum Stand der Technik. Bevorzugt sind polyklonale Antikörper als auch monoklonale Antikörper, die die dimere, d.h. die Tumorform der Pyruvatkinase binden.

Die quatrinäre Struktur eines Eiweißes betrifft die räumliche Anordnung der Untereinheiten des Eiweißes.

Die "normale M2-PK" besitzt eine tetramere Struktur (quatrinäre Form). Die M2-PK von gesunden Personen unterscheidet sich von der "Tumor M2-PK". Die "Tumor M2-PK" besitzt eine dimere Struktur.

Polyklonale Antiköper und monoklonale Antikörper sind gegen diese speziellen Formen (Tumor M2-PK dimer oder M2-PK tetramer) durch klassische Immunisierungsmethods, als auch durch Hybridoma-Methodik (Köhler-Mielstein-Technologie) erhältlich.

So ist die Tumorform der M2-PK zu reinigen und als Antigen zu verwenden. Eine andere Möglichkeit ist es, gentechnisch exprimierte M2-PK auch die Tumor-Form zu kaufen und diese für die Immunisierung zu verwenden. Ein anderer Weg ist es, spezielle Fragmente sprich Aminosäuresequenzen zu synthetisieren und diese bei der Immunisierung zu verwenden.

**Tumor M2-PK Antikörper**

Bevorzugt zum Aufsprühen auf die Nitrozellulosemembran ist ein monoklonaler Antikörper der Maus (Klon PATAM3AT, IgG1), Dieser Klon ist durch Hybridisierung von Myelomazellen mit B-Lymphozyten der Maus generiert worden. Als Immunogen diente rekombinante humane Tumor M2-PK aus E. coli mit den Aminosäuren 1-531, Der Antikörper ist u.a. bei der Firma Prospec (Esst Brunswick, USA) erhältlich. Wahlweise kann wie hierin beschrieben ein monoklonaler Antikörper der Maus (Klon AT1 E3, IgG1) verwendet werden. Als Immunogen diente rekombinante humane Tumor M2-PK. Der Antikörper ist u.a. bei der Firma Novus Biologicals (Littleton, USA) erhältlich. Besonders bevorzugt für die Kopplung am Gold ist ein monoklonaler Maus-Antikörper (Klon 1 E3, IgG1). Als Immunogen diente rekombinante, humane Tumor M2-PK mit der Aminosäureseq 47-574. Der Antikörper ist u.a. bei der Firma Novus Biologicals (USA) erhältlich. Wahlweise kann ein im Schaf erzeugter polyklonaler Antikörper (Ig-Fraktion) der Firma Randox (United Kingdom) verwendet werden. Als Immunogen diente rekombinante humane Tumor M2-PK aus E.coli.

**Hämoglobin Antikörper**

Bevorzugt zum Aufsprühen auf die Nitrozellulosemembran ist ein monoklonaler Antikörper der Maus (Klon M1202100, IgG1). Der Antikörper wurde erzeugt, indem mit humanem Hämoglobin immunisiert wurde. Der Antikörper ist u a, bei der Firma Thermo Scientific (Rockford, USA) erhältlich. Alternativ kann wie hierin beschrieben ein monoklonaler Antikörper gegen humanes Hämoglobin eingesetzt werden. Dieser Antikörper aus dem Klon 7202 SPR-5 hat eine Affinitätskonstante von $1 \times 10^{-10}$ l/Mol und einen isoelektrischen Punkt von 5,8. Er kann über die Firma Medix (Finnland) bezogen werden. Für den an Gold gekoppelten Hämoglobin Antikörper wurde der monoklonale Mausantikörper (Klon HB11-2312) benutzt. Als Immunogen wurde gereinigtes humanes Hämoglobin benutzt, Der Antikörper kann bei der Firma Thermo Scientific (Rockford, USA) bezogen werden.

Eine der wichtigen Bedingungen zur Auswahl des Antikörpers ist, dass diese nicht mit anderen Bestandteilen des Stuhls, insbesondere nicht mit anderen Pyruvatkinase-Isoenzymen (z.B. M1-PK, M2-PK (tetramere Form) L-PK, R-PK), kreuzreagieren dürfen.

Die oben genannten Hämoglobin Antikörper sind besonders geeignet zum Binden an eine Membran (Festphase), in bevorzugter Form Nitrozellulose. Die oben genannten Antikörper kreuzreagieren nicht mit Hämoglobin vom Schwein, Pferd, Schaf und Rind. Die oben genannten Hämoglobin Antikörper eignen sich vorzugsweise zum Binden an Latex- oder Nano-Partikel - bevorzugt Goldkolloide - (Flüssigphase). Sie kreuzreagieren nicht mit Hämoglobin vom Schaf, Pferd, Rind oder Schwein. Die oben genannten Antikörper binden Hämoglobin A0 und A1 gleich gut, A2 teilweise mit geringerer Bindungskonstante und AS teilweise mit sehr schlechter Bindungskonstante. Festphase-Antikörper werden bevorzugt 1:1 w/w gemischt. Flüssigphase-Antikörper werden bevorzugt 1:1 w/w gemischt.

Detergenz 1 (*CHAPS (3-[(3-Cholamidopropyl)dimethylammonio]-1-propansulfonat, 10 mM (Sigma)),* Detergenz 2 = Sodium Dodecyl Sulfate (SDS) 0,01% - 0,1 %), z.B. von, Detergenz 3 = Lauryldimethylamine Oxide (10 mM-50mM), z.B. von Biochrom oder Mischungen hiervon.

Die Entnahmevorrichtung zur Durchführung des Hämoglobintests enthält einen der oben beschriebenen Puffer (1,5-2,5 ml). Die zu testende Person sticht mit der Dosierspitze in den Stuhl und überführt die Stuhlprobe in das mit dem entsprechenden Puffer gefüllte Röhrchen.

Die Entnahmevorrichtung für den Tumor M2-PK-Test, die der zu testenden Person vom Arzt übergeben wird, enthält keinen Puffer. Der Patient sticht mit der Dosierspitze in den Stuhl und überführt die Stuhlprobe in das leere Röhrchen.

Das erfindungsgemäße Testkit enthält weiterhin eine Testkassette, in der die nach dem Stand der Technik verbesserten beiden Immunoassays durch Lateral-Flow-Technologie durchgeführt werden. Die Testkassette enthält zu diesem Zweck zwei, vorzugsweise runde, Aussparungen zum Auftragen der Stuhlproben sowie zwei, vorzugsweise längliche, Aussparungen zur Ablesung der Testergebnisse.

Die Testkassette enthält in ihrem Inneren als stationäre Phase Nitrozellulose folgender bevorzugter Kapillarflussrate von 135 sec/4 cm.

Bei den Untersuchungen im Rahmen der Entwicklung des erfindungsgemäßen Testkits hat sich herausgestellt, dass die Nitrozellulose einen starken Einfluss auf die Streuung der Ergebnisse hat. Diese Streuung ist besonders groß bei frisch hergestellter Nitrozellulose, wird aber geringer durch eine drei- bis sechsmonatige Lagerung (Reifung). Bei der im Rahmen der vorliegenden Erfindung verwendeten Nitrozellulose handelt es sich um solche, die drei bis sechs Monate gelagert wurde, bevor sie weiteren Tests unterzogen wurde. Durch die Verwendung derartiger "gereifter" Nitrozellulose konnte eine deutliche Reduzierung der Ergebnisstreuung erzielt werden. Insbesondere konnte erreicht werden, dass zwischen verschiedenen Chargen der hergestellten Testkits deutlich geringere Varianten auftreten als bei "frischer" Nitrozellulose.

Die Antikörper sind an Kolloide, bevorzugt Goldkolloide oder Latexkolloide gekoppelt, bevorzugt an Goldkolloide mit 20 Nanometern Durchmesser.

Die stationäre Phase = Nitrozellulose (bevorzugt mit der Kapillarflussrate von 90 sec/4cm) enthält Hämoglobin Antikörper 1, Hämoglobin Antikörper 2, bevorzugt ist ein Mischungsverhältnis wie oben angegeben.

Die stationäre Phase = Nitrozellulose (bevorzugt mit der Kapillarflussrate von 135 sec/4cm) enthält ferner Tumor M2-PK Antikörper (aus Klon P1F3), Tumor M2-PK Antikörper (aus Klon P5A1), bevorzugt eine Mischung aus Antikörpern der Klone P1F3 und P5A1 in folgendem Mischungsverhältnis Volume 1:3,5 w/w.

Antikörper aus Klon P1F3 und Klon P5A1 eignen sich besonders als Fängerantikörper (stationäre Phase) zum Binden an eine Membran, vorzugsweise Nitrozellulose. Tumor M2-PK Antikörper aus Klon P1A6 eignen sich bevorzugt zum Binden an Goldkolloiden oder Latexkolloiden, bevorzugt Goldkolloiden mit 80 Nanometern Durchmesser.

Geräte und Protokolle zur Testkitherstellung, insbesondere zur Herstellung der beiden Teststreifen:

Einschweißgerät - Folieneinschweißgerät (der Firma Kopp) Verpackungssysteme Reichenbach

Siegeltemperatur 60°, Type MSC 440 Watt

Biodot-Gerät

2. Biodot-Gerät

Cutter der Firma Zeta Corporation, www.zetacorporation.com Gerätetyp GC1800-081101

Gerät ist CE-gekennzeichnet

Protokoll für Laminieren und Schneiden der Nitrozellulosemembran Anzahl der laminierten M2-PK-Karten

Geräteeinstellung und Bedienung Laminiergerät Matrix 2210 der Firma Kinematic

Protokoll für Laminieren und Schneiden der Nitrozelluiosemembran Ge-räteeinstellung, Bedienung Laminiergerät Matrix 2210 Durchführung Geräteeinstellung und Bedienung

Geräteeinstellung und Bedienung Schneidegerät GSI-800 der Firma Zeta Corporation

Fertigen von Kassetten und Einräumen der SchaBo M2-PK-Quick-Kits

Fertigen von Testkassetten

Einlagerung der Teilfertigprodukte in Kühlschränken

Qualitätskontrolle

Die Temperatur und Luftfeuchtigkeit der Räume, in denen sich die Herstellung befindet, wird aufgezeichnet

Klimagerät FUJITSU DG Inverts für die Raumklimatisierung und Konstanthaltung der Luftfeuchtigkeit und der Temperatur_ Die Einhaltung der Lufttemperatur und Luftfeuchtigkeit ist von besonderer Bedeutung für die Qualitätssicherung der Testmembranen.

Wahl der Betriebsart / Einstellung des Thermostats 1 Einstellen der Gebläsedrehzahl

Kritische Erfolgsfaktoren zur optimalen Abstimmung der beiden Test-streifen in der Kombikassette sind im Besonderen:

a) Heidelberger-Kurven für Hb-Bestimmung

b) Heidelberger-Kurven für M2-PK-Bestimmung

Überraschenderweise wurde gefunden, dass wenn man dem Extraktions-puffer M2-PK 6,7‰ Ochsengalle (natürliches Netzmittel, gereinigt der Firma Schmincke, Bestell-Nr.: 50031, Schmincke, Erkrath, Deutschland) als Endkonzentration beimischt, sich besonders gute, reproduzierbare immunchromatographische Ergebnisse erzielt werden.

Überraschenderweise wurde gefunden, dass wenn man dem Extraktions-puffer für Haemoglobin 2,8‰ Ochsengalle als Endkonzentration beimischt, sich besonders gute, reproduzierbare immunchromatographische Ergebnisse erzielt werden.

Zur-technischen synchronen Bestimmung der beiden Analyten in der Kombi-Kassette

Optimierung der "Zeitversetzung" des Chromatographie-Startzeitpunktes Der Anwender startet die Chromatographie durch Auf-tropfen von M2-PK¬Stuhlproben-Extrakt in das linke Probenfenster der Kassette (stellt dann den Timer auf 5 Minuten). Das Auftropfen des Hb-Stuhlprobenextraktes erfolgt nach 1 Minute nach Auftropfen des M2-PK-Stuhlprobenextraktes in das rechte Probenfenster der Kassette. D.h. die beiden Chromatographien werden zeitversetzt gestartet. Das Ergebnis der beiden Tests wird jedoch nach Ablauf der 5-minütigen Stoppzeit abgelesen.

Chromatographie im Besonderen immunchromatographische Testverfahren, sind - wie der Name sagt - von der Zeit, als auch von Bindungsaffinitäten (die Bindung der Antikörper an die Antigene in Flüssig- und Festphase, aber auch die vielfältigen Kinetiken innerhalb der Nitrozellulosemembran) im Wesentlichen abhängig, d.h. sie sind vielfältigen Kinetiken unterworfen.

Testergebnisse, die später abgelesen werden, sind ungültig! (Dies ist alles in der Testbeschreibung des Kombi-Tests angegeben). Die Bildung der rosaroten Testlinie im Testbereich des Nitrozellulosest-reifens der Testmembran ist biophysikalisch das Ergebnis einer komplexen Agglutinationsreaktion (vielfältige Kinetiken und Affinitäten spielen hier eine besondere Rolle; siehe auch die oben näher zu beschreibende Heidelberger-Kurve). Zur erfindungsgemäßen parallelen, d.h. synchron im technischen Sinne Abarbeiten oder Bestimmen der erfindungsgemäßen Analyten 1. Start der "Chromatographie" M2-PK gefolgt von einem zweiten Start der Chromatographie Hb

Die synchrone Bestimmung im technischen Sinne ist eine Herausforderung, da die immunchromatographische Bestimmungen sehr komplex sind.

Viele Parameter sind von besonderer Wichtigkeit zur Lösung der viel-fältigen, erfinderischen Aufgaben:

(alle Aufgaben wurden bei der ScheBo Biotech AG gelöst).

Diese Aufgaben wurden erfindungsgemäß gelöst durch z.B. durch Ermittlung besonderer/essentieller Wirkpaarungen:

1. Bindungskonstante/Affinität der einzelnen Antiköper an die jeweiligen Stoffe (Proteine), die erfindungsgemäßen Analyten. Kritisch ist auch die Zusammensetzung des "getrockneten" Goldes und die "Freisetzung" des Goldes vom PAD-Release, d.h. die Auflösung der getrockneten Goldpartikei (in einer Glasfasermatrix/getrockneten Zu-ckermatrix befindend) durch das Zutropfen des Extraktionspuffers, welcher den entsprechenden Analyten enthält (Stuhlprobenextrakt).

Zur Herstellung des erfindungsgemäßen Testkits beinhaltend eine Doppelkassette zur immunchromatographischen Bestimmung zweier Analyten.

Um dieses zu bewerkstelligen, testeten wir die immunchromatografische Bestimmung der beiden Analyten erst in einem so genannten Dipstick-Format. Dieses Dipstick-Format ist wesentlich weniger kompliziert, da es nicht das Conjugate-Release-PAD beinhaltet.

Folgende Aufgaben und Herausforderungen waren erfindungsgemäß zu lösen:

Die erfindungsgemäßen technischen Lösungen waren fast alle "nicht naheliegend".

Generelle Anmerkungen:

Für die Herstellung der Teststreifen ist es von besonderer Wichtigkeit, die Luftfeuchtigkeit zwischen 40 - 60% bei 18°C bis 25°C zu halten,

Durch Testreihen, die in der Abteilung F&E bei der ScheBo-Biotech AG durchgeführt wurden, wurde eine optimale Luftfeuchtigkeit von 35% bei 20°C erfinderisch ermittelt.

Aufgaben/Herausforderungen:

1 a. Langsam fließende Flüssigkeit in der Membran oder sogar Stopp/ Unterbrechung des chromatographischen Flüssigkeitsflusses.

1 b. Die Probe in der Flüssigkeit stoppt in der Mitte (in Laufrichtung der Membran) des Membranstrips.

Ist die ausgewählte Membran zu langsam in der Flussgeschwindigkeit für die Applikation?

Wurde die Membran hydrophob?

Wurde das richtige Wick verwendet?

Die Nitrozellulosemembran enthält "oberflächenaktive Substanzen" und "Benetzungsreagenzien"

Die Testmembran besitzt eine hohe "Background-Färbung" (diese Back-ground¬Färbung wurde durch erfindungsgemäße, nicht naheliegenden Lösungen gelöst)

Es ist zu klären, ob spezielle Blocking-Reagenzien einzusetzen sind.

Die Abgabe des Goldes auf die Nitrozellulosemembran optimal und komplett einstellen (durch erfindungsgemäße, nicht naheliegende Lösung gelöst)

Die Flüssigkeitsfront ist nicht gradlinig (durch erfindungsgemäße, nicht naheliegende Lösung gelöst).

Testlinie ist zu dick oder "fuzzy", "verschmiert" (durch erfindungs-gemäße, nicht naheliegende Lösung gelöst)

Ein viel zu schwaches Signal an der Testlinie wird reproduzierbar beobachtet/gefunden (durch erfindungsgemäße, nicht naheliegende Lösung gelöst).

Die Testlinie zeigt falsch-positive/falsch-negative Ergebnisse (durch erfindungsgemäße, nicht naheliegende Lösung gelöst)

Das erfindungsgemäße Testkit enthält Metallkolloide, d. h. Gold-kolloid. Auch beschrieben sind z.B. Latexpartikel, auch Nanopartikel. Latexpartikel können gefärbt auch mit Fluoreszenzfarbstoffen versehen sein und unterschiedliche Größe besitzen (Größe heißt im Mikrometerbereich aber auch im Nanometerbereich)

Auch magnetische Partikel (Auslösung Quantifizierung mittels spezieller Lesegeräte (Reader) möglich).

Die großen technischen Herausforderungen bezogen sich speziell auf die Qualitätssicherung. Erstellung von good-manufacturing-Protokollen (durch erfindungsgemäße, nicht naheliegende Lösung gelöst).

Ziel/Aufgabe: Erreichung einer optimalen, kleinen intee- bzw. inter-Assay-Varianz Besonders wichtig: sehr gute Lot-to-lot-Varianz (d.h. eine möglichst kleine Lot-to-Lot¬Varianz).

Diese kleine Lot-to-Lot-Varianz ist sicherzustelien im Rahmen einer Qualitätssicherung in den erfindungsgemäßen Herstellungsverfahren (durch erfindungsgemäße, nicht naheliegende Lösung gelöst)

Herstellung einer großen Anzahl, mehr als 100.000 Testkits mit gleicher Lot¬Nummer, gleichem Haltbarkeitsdatum (durch erfindungsgemäße, nicht naheliegende Lösung gelöst).

Herausforderung: Sicherstellung extrem guter Qualität - Vorteil für den Kunden (durch erfindungsgemäße, nicht naheliegende Lösung gelöst).

**Agglutination - Definition**

Agglutination bezeichnet in der Medizin die Verklebung bzw. Verklumpung von Zellenerregern usw.

Agglutinationsreaktionen werden in der Labordiagnostik dazu genutzt, quantitative Aussagen zu machen. Die Verklumpung wird gemessen mittels nephelometrischer Bestimmungsmethoden.

Die rote Testlinie entsteht in komplizierter Kinetik durch eine Agglutinationsreaktion. In der dreidimensionalen Struktur der Nitrozellulose des Teststreifens findet eine Verklumpung statt. Diese "Verklumpung" ist mit bloßem Auge als rosa Linie erkennbar.

Diese rosarote Linie ist das Ergebnis einer Substanzerkennung durch Antigen¬/Antikörper-Reaktion. Diese Antigen-/Antikörper-Reaktion ist vielfältigen Effekten unterworfen, Einer dieser Effekte ist der so genannte Hook-Effekt (auch als high¬dose-Hook-Effekt bezeichnet), der in falsch-niedrigen Bestimmungen von Analyten, die in sehr hoher Konzentration von Probenlösungen vorkommen (d.h. hohe Konzentrationen des Analyten können im besonderen Falle falsch-negative Messsignale vortäuschen).

Sobald die Analyt-Konzentration zu hoch ist, können die Antikörper-Bindungsstellen mit Analyt belegt sein und die zusätzlichen Analyt-Moleküle werden nicht mehr im Rahmen der Bindungskurve ermittelt. Es kommt zu falsch-niedrigen Messwerten.

Eine technische Aufgabe war die Vermeidung des Hook-Effektes. Durch parallele Messungen von verschiedenen Verdünnungen einer Probe kann das Vorliegen eines high-dose-Effektes bemerkt und die Messung entsprechend korrigiert werden (die Aufgabe des technischen Problems wurde durch erfindungsgemäße, nicht naheliegende Lösungen gelöst).

Das in den Ansprüchen beschriebene erfindungsgemäße Testkit weist im Gegensatz zu Ausführungsformen aus dem Stand der Technik keinen High Dose Hook Effekt auf. Dies wurde durch Tests mit Proben, mit Tumor M2-PK Konzentrationen, die den Cut-off um das mehr als 200-fache überstiegen (bis zu 160.000 ng/ml), überzeugend nachgewiesen.

Generell unterliegen die immunbioanalytischen Nachweisverfahren, speziell immunchromatographische Verfahren, folgenden Störungen:

1. Störungen durch Kreuzreaktivität und unspezifische Bindungen
2. Störungen durch Matrixeffekte
3. Störungen durch "Anti-Animal-Antibodies'°
4. Störungen durch endogene Bestandteile der Probe
5. Störungen durch Heterophile und andere quer-vernetzende Störer

(Diese Störungen 1-5 konnten durch erfindungsgemäße, nicht naheliegende Lösungen gelöst werden)

Von Besonderheit war die Verwendung eines speziellen (von der ScheBo Biotech AG hergestellten) LowCross-Buffers. Dieser enthält verschiedene Detergenzien, Proteine, polyklonale Antikörper, oberflächenaktive Substanzen als auch Substanzen, die die Oberflächenspannung verändern.

All dieses zusammen mit den zuvor beschriebenen Reagenzien, Substanzen usw. des erfindungsgemäßen Testkits ergibt eine optimale Abstimmung der Wirkpaarungen im technischen Sinne. Dieses alles erlaubt die synchrone Bestimmung der beiden Analyten in einer Kombi-Testkassette.

Überraschenderweise wurde gefunden, dass zur Lösung der gemeinsamen, synchronen (im technischen Sinne) Bestimmung der beiden Analyten auf den Teststreifen die Feuchtigkeitskontrolle (Humidity control) und die Entgasung der Flüssigkeiten beim präzisen Dispensieren der Antikörperflüssigkeiten und des kollodialen Goldes von ausschlaggebender Bedeutung war.

Kreatives, aber auch systematisches Arbeiten war nötig, um das erfindungsgemäße Testkit bereitstellen zu können.

Aufwendige Testreihen war notwendig zur Erkennung von Wirkpaarungen und deren gemeinsame, optimale Abstimmung.

Dieses ist eine Grundvoraussetzung, um einen robusten, reproduzierbaren und verlässlichen Test in Form eines Schnelltests (Plattform oder/und Point-of-Care¬Tests) zu entwickeln.

Die extrem große Variabilität der Proben des verfügbaren Materials (Stuhlprobanden), Reagenzien und Antikörper jeweils zwei Pärchen müssen untereinander abgestimmt werden, um reproduzierbare Ergebnisse nach und nach zu erhalten.

Beim Design und bei der Entwicklung einer solch komplexen Plattform stößt man auf zahlreiche physikalische und chemische Phänomene, die das Testergebnis unerwartet beeinflussen können.

Eine andere Ausführungsform des wie hierin beschrieben ist die Verwendung, nach Austestung "aller" optimaler, essentieller Wirkpaarung, die qualitative, semiquantitative und quantitative Bestimmung der beiden Analyten (Biomarker mittels anderer immunchemischer Testprinzipien z.B. ELISA) Agglutination bevorzugt die Verwendung von Nanopartikeln zur Auslösung der Agglutination in einer Flüssigphase. Turbometrische, nephelometrische Messtechnik sind nach dem Stand der Technik ermöglicht die Generierung von Messdaten. Diese Messdaten lassen sich mittels entsprechender Computersoftware verarbeiten. Mittels einer "internen" Eichkurve (auch chargenabhängig) ist z.B. eine Bestimmung der beiden erfindungsgemäßen Analyten möglich. Auch vielfältige grafische Darstellungen z.B. Log-Log, Log¬dekadische Darstel-ung .... möglich) Hierin beschrieben ist die statistische Auswertungen, z.B. Algorithmen aus den Bereichen Fuzzy-Logic-Analysen, Klassifikation sowie Muster¬und Bilderkennung, explorative Datenanalyse, Datenvisualisierung, robuste und computergestützte Statistik, Data-Analysis (IDA) and Evolving Systems (ES)

Data-Mining und explorative Datenanalyse, Fuzzy-Systeme, neuronale Netze, evolutionäre Algorithmen. Bevorzugt sind die Algorithmen von Herrn Prof. Dr. Frank Klawonn. Prof. Klawonn hat gute Auswertestrategien, die eine Datenverlässlichkeit verbessern. Prof. Klawonn ist der Direktor des Instituts für angewandte Informatik. Weiter hierin beschrieben ist eine Schnittstelle zu WLAN Auch zeitliche Verläufe von Patienten sind möglich Eine individuelle Patientendatenreihe ist möglich zur Auswertung

Andere Apparate, Vorrichtungen usw. zur Messung der beiden Analyten sind denkbar.

Andere Ausführungsformen Plattformen "ELISA" sind denkbar

Verschiedene Arrays: flüssig (Luminex) oder fest Phasen (Arrays) sind möglich. Sämtliche immunchemische Nachweisverfahren sind denkbar

**Agglutination**

Überraschenderweise wurde gefunden, dass die Tröpfchengröße (Volumen im Nanoliter-Bereich) und der Abstand dieser Tröpfchen ausschlaggebend ist.

Die Tröpfchengröße und der Abstand zwischen diesen Tröpfchen ist von besonderer Bedeutung. Es wird somit keine "Linie" dispergiert sondern Einzeltröpfchen. Die Kapillarkräfte der Nitrozellulosemembran saugen in Bruchteilen von Sekunden Tröpfchen auf und lassen eine Linie entstehen. Die in der Flüssigkeit befindlichen Antikörper verteilen sich in der dreidimensionalen Struktur der Nitrozellulose.

Überraschenderweise wurde gefunden, dass es für die Erreichung einer sehr präzisen Linie der "Trocknungsgrad" entscheidend ist.

Überraschenderweise wurde auch gefunden, dass die Hydrophobizität sehr, sehr wichtig ist. Zur Gewährleistung, dass die zu verwendende Nitrozellulosemembran trocken ist, wurde diese zuvor in einseitig beschichteten Aluminiumfolien, die keine Feuchtigkeit durchlassen, eingeschweißt, geöffnet, mit Antikörpern versehen und sofort wieder in die Aluminiumfolien plus Trocknungsmittel eingeschweißt. Diese Herstellungsschritte waren überraschenderweise wichtig und erfolgten im 6-Minuten¬Takt.

Überraschenderweise war es verwunderlich, dass um einen konstanten Fluss über die Membran zu gewährleisten, der Aufbau des Teststreifens in der richtigen Reihenfolge erfolgte.

In der Reaktionszone der Teststreifen (Testergebnislinienregion) entstehen die entsprechenden Linien (rosarote Linien)

Die farbige Testergebnislinie (ein positives Ergebnis) ist das Ergebnis einer Antigen¬Antikörper Reaktion und der ermittelten "Heidelberger Kurve" die Messsignal in Abhängigkeit von Antikörperüberschuss und Antigenüberschuss zeigt. Ziel ist es, den Äquivalenzbereich gemäß der "Heidelberger Kurve" zu nutzen.

Die optimale Nutzung der Antigen-Antikörperreaktion, d.h. die Abstimmung der stationären Antikörper zu den in der Flüssigphase befindlichen antikörpergekoppelten Goldpartikel im Äquivalenzbereich des M2-PK-Teststreifens ergibt ein optimales Signal für M2-PK auf dem Teststreifen.

Die optimale Nutzung der Antigen-Antikörperreaktion, d.h. die Abstimmung der stationären Antikörper zu den in der Flüssigphase befindlichen, antikörpergekoppelten Goldpartikeln im Äquivalenzbereich des Hb-Teststreifens ergibt ein optimales Signal für Hb auf dem Teststreifen.

Die optimale Nutzung der Antigen-Antikörperreaktion in dem Äquivalenzbereich des einen M2-PK-Teststreifens und in dem Äquivalenzbereich des Hb-Teststreifens war die erfinderische Aufgabe.

Die erfinderische Aufgabe wurde gelöst durch die optimale gemeinsame Abstimmung von Wirkpaarungen, im Besonderen durch die richtige Wahl der Antikörper, die richtige Wahl von Detergentien usw., aber im Besonderen durch die reproduzierbare, hochpräzise Dispensierung der Antikörperlösungen auf den beiden Nitrozellulosetestmembranen.

Dieses ermöglichte den synchronen Nachweis der beiden Analyten in Stuhlproben von Probanden. Alle diese Wirkpaarungen und die technischen Lösungen dazu sind in der Anmeldung beschrieben. Hierzu waren oft nicht nahe liegende Lösungen, auch glückliche Zufälle führten zum positiven Ergebnis.

Das Hämoglobinteströhrchen und das M2-PK-Röhrchen müssen innerhalb von 48 Stunden in der Arztpraxis abgegeben werden. Die Dosierspitze mit der Stuhlprobe wird in ein mit oben beschriebenem Puffer gefülltes Röhrchen überführt. Sowohl das Röhrchen für den M2-PK-Test als auch das Röhrchen für den Hämoglobintest werden kräftig geschüttelt.

Die vorbereiteten Stuhlprobenextrakte werden unmittelbar nacheinander auf die beiden Aussparungen aufgetragen. Nach 5-10 Minuten wird anhand der markierten Linie abgelesen, ob einer oder beide der gemessenen Biomarker positiv sind.

Entscheidend für die sichere Bestimmung eines malignen Geschehens im Gastrointestinaltrakt ist die Wahl des sogenannten "Cut-Offs". Der Cut-Off markiert die minimale Konzentration des Analyten in der Probe, die ein positives Ergebnis anzeigt. Der Cut-Off des Hämoglobintests beim erfindungsgemäßen Testkit liegt zwischen 18 und 38 $\mu$g Hämoglobin pro Gramm Stuhl, bevorzugt bei 24 $\mu$g Hämoglobin pro Gramm Stuhl. Der Cut-Off bei Tumor M2-PK liegt zwischen 3 und 6 U/ml Stuhlextrakt, erfindungsgemäß 4 $\pm$ 1 U/ml.

Die Nachweisgrenze beträgt 150 Nanogramm Hämoglobin/ml Stuhlextrakt.

Die Auswertung des Testergebnisses und Zuordnung zu den Risikostufen, wie sie beispielhaft in Figur 10 dargestellt ist kann manuell, semiautomatisch oder vollautomatisch erfolgen. Beispielsweise kann eine erfindungsgemäße Testkassette manuell in eine Auslesevorrichtung eingespannt werden, die mittels einer Fotozelle oder Kamera eine Auswertung vornimmt und das Ergebnis dann auf einem Monitor darstellt. Bevorzugt erfolgt die Darstellung in Farbe (Risikoampel). Besonders bevorzugt wird aus Sicherheitsgründen eine parallele Darstellung in anderer Form (z.B.: Risikostufe 1-4, o.ä.).

Selbstverständlich kann die Testkassette auch so ausgestaltet sein, dass eine vollautomatische Auswertung möglich ist, z.B. durch Nachweis der Goldpartikel auf elektrochemischem Wege oder durch eine optische Aufnahme (Scannen) des Testkits.

**Beispiel 2:**

Die erfindungsgemäße Kombi-Schnelltestkassette enthält nicht den am Markt befindlichen Lateralflowteststreifen zur Bestimmung der M2-PK, sondern einen neuen, verbesserten, erfindungsgemäßen Lateralflowteststreifen zur Bestimmung der M2-PK (Tumor M2-PK plus), z.B Sartorius.

Des Weiteren enthält die Kombi-Schnelltestkassette einen nicht am Markt befindlichen Lateralflowteststreifen zur Bestimmung von Hämoglobin, sondern einen neuen, verbesserten, erfindungsgemäßen Lateralflowteststreifen zur Bestimmung von Hämoglobin (iFOBplus), z.B Sartorius.

Die Erfindung betrifft im Besonderen den Kombi-Schnelltest und Bestandteile sowie alle nötigen Reagenzien zur synchronen Durchführung des immunchromatographischen Nachweisverfahrens. Synchron in der sprachlichen Bedeutung von gleichzeitig aber auch synchron in der technischen Bedeutung der gemeinsamen, optimalen Abstimmung der chromatographischen Bedingungen zum Nachweis der beiden Biomarker M2-PK und Hämoglobin in einer Kombi-Schnelltestkassette.

**Nachweis Reagenzien:**

Verschiedene Nachweisreagenzien können verwendet werden: Latexkugeln, Colloidal-Goldpartikel, Colloidal-Silberpartikel usw. Eines der wichtigsten Eigenschaften dieser Partikel ist, dass die Population monodispers mit einer konstanten sphärischen Größe sein muss.

Erfindungsgemäß verwendet sind Goldpartikel. Die Herstellung colloidaler Goldpartikel ist im Prinzip bekannt. Üblicherweise wird eine Lösung enthaltend $Au^{3+}$ unter schnellem Rühren chemisch reduziert, so dass atomare Goldpartikel ausfallen, welche sich im Laufe der Zeit aggregieren. Die Aggregation kann durch Stabilisierungsmittel verhindert werden. Durch Wahl des richtigen Zusatzmittels kann die Größe der gebildeten Colloide eingestellt werden. Als $Au^{3+}$-Quelle wird häufig $H[AuCl_4]$ verwendet. Als Reduktionsmittel kann Natriumcitratlösung, Natiumborhydrid oder Hydrochinon verwendet werden. Zur Stabilisierung werden häufig Schwefelverbindungen (wie beispielsweise Alkanthiole) verwendet. Lösungen enthaltend Goldpartikel sind von verschiedenen Quellen erhältlich.

**Polymerzusammensetzung und Proteinbindung**

Zur Herstellung von Lateral-Flow-Tests verwendet man Nitrozellulose, Polyvinyliden Fluorid, ladungsmodifiziertes Nylon, Polyethersulfon.

Die Polymeroberflächengröße ist abhängig von Porengröße, Porosität, Dicke und weiteren strukturellen Charakteristika.

Die Oberfläche nimmt nicht-linear mit der Porengröße zu, sondern steigt linear mit der Dicke und steigt nicht-linear mit der Porosität. Die Proteinbindung an einen gegebenen Oberflächenbereich ist abhängig von der Dichte des Proteins, seiner Struktur und seinem Stokes-Radius (effektiver Durchmesser). Ferner ist die Bindung des Proteins am Polymer abhängig vom pH-Wert und der Immobilisierungslösung.

Im erfindungsgemäßen Kombi-Schnelltest wurden bevorzugt für das so genannte Sample-Pad Glasfaser, für die Testmembran Nitrozellulose und für das Absorbant-Pad Zellulose verwendet.

```
Konjugat/Sample-Pad = 22 mm (+/- 0.2 mm) x 5 mm (+/-0.2
mm)

Nitrozellulose-Membran  = 25 mm (+/- 0.2 mm) x 5 mm (+/-
0.2 mm)

Absorbant Pad = 16.5 mm (+/- 0.2 mm) x 5 mm (+/- 0.2 mm)

     Die Breite der Testmembran = 4 mm (+/- 0.2 mm)
```

Kapillare Flussrate: normalerweise 1-6 cm/min.; erfindungsgemäß 3,74 cm/min.

**Nachweisgrenze**

Der cut-off von 4 U M2-PK/ml wurde aufgrund unterschiedlicher Studien, die den M2-PK-Stuhltest ELISA untersuchten, gefunden. Die erfindungsgemäß chromatographischen Bedingungen z.B. des cut-off-Wertes des erfindungsgemäßen Kombi-Schnelltests wurde aufgrund des vorher ermittelten cut-off-Wertes des M2-PK-Stuhltests ELISA festgelegt.

**Die kapillaren Flussraten für Nitrozellulosemembranen**

Die analytische Sensitivität nimmt mit der kapillaren Flusszeit ab d.h. die Nitrozellulose mit der langsamsten kapillaren Flusszeit ergibt die höchste analytische Sensitivität. Kapillare Flusszeiten liegen zwischen 240 - 75 sec/4 cm. Erfindungsgemäße kapillare Flussrate 120 sec/4 cm.

**Liste der erfindungsgemäßen Komponenten und erfindungsgemäßen aktiven Bestandteile**

| Bestandteil | Reagenzien | Menge |
|---|---|---|
| Fanglinie<br>1. Antikörper<br>2. Antikörper | Monoklonaler Anti-Maus Anti-M2-PK-Antikörper Maus-Anti-Hämoglobin-Antikörper | 0.20 - 130 $\mu$g Protein; 0.5 - 3 $\mu$g/cm Protein |
| Fanglinie<br>Konjugat | Colloidale Goldpartikel, an denen die erfindungsgemäßen Anti-M2-Antikörper bzw. Anti-Hämoglobin-Antikörper gekoppelt wurden. | 0.01 - 0.07 $\mu$g Protein |
| Kontrolllinien<br>Antikörper | Spezielle AntiMaus-IgG-Antikörper, z.B. von der Firma Jackson Immunochemical (USA). | 0.20 - 1.30 $\mu$g Protein<br>0.05 - 1 $\mu$g/cm Protein |

**Extraktions-/Laufpuffer**

| Reagenzien | Menge |
|---|---|
| Triton X-100 p. A. | < 0.50% |
| Sodium azide | < 0.05% |

Bei den Untersuchungen im Rahmen der Entwicklung des erfindungsgemäßen Testkits wurde überraschenderweise gefunden, dass sowohl die klinische Sensitivität und Spezifität, aber auch die chemisch-analytische Empfindlichkeit deutlich erhöht wird, wenn die Laufflüssigkeit im immunchromatografischen Testprinzip einen pH-Wert zwischen 5 und 6 aufweist, vorzugsweise einen pH-Wert von 5,7. Dieser Befund ist besonders überraschend, da nach dem Stand der Wissenschaft zu Eigenschaften von Proteinen diese bei einem pH-Wert von 5,7 partiell denaturiert vorliegen sollten. Es ist daher überraschend, dass in diesem Bereich eine Verbesserung von Sensitivität, Spezifität, aber auch Empfindlichkeit erreicht wird. Alle bislang auf dem Markt befindlichen Testkits zur immunchromatografischen Bestimmung von Hämoglobin weisen einen pH-Wert von 7 bis 7,5 des Laufpuffers auf. Eine abschließende wissenschaftliche Erklärung hierfür kann bislang nicht geliefert werden. Möglicherweise, ohne sich an diese Erklärung binden zu wollen, kommt es zu einer partiellen Denaturierung in der Weise, dass die Epitope der Analyten für die verschiedenen Antikörper besser zugänglich sind. Zur Durchführung dieser Ausführungsform der Erfindung mit einer Laufflüssigkeit mit einem pH von 5-6, insbesondere 5,7 wird ein entsprechender Puffer benötigt, nämlich ein Acetatpuffer (pH ≈ 5,7), welcher vorteilhafterweise noch 0,1% Albumin als Stabilisator enthält. Derartige Puffersysteme sind dem Fachmann vertraut und bedürfen an dieser Stelle keiner weiteren Vertiefung.

**Beschreibung der Figuren**

**Figur 1** Schematische Ansicht eines Teststreifens in der Plastikkassette eines Lateral-Flow-Schnelltests.

| 1 | Sample Port | Probeneingang |
|---|---|---|
| 2 | Test Line | Testlinie |
| 3 | Control Line | Kontrolllinie |
| 4 | Housing | Verpackung |
| 5 | Sample Pad | Probekissen |
| 6 | Conjugate Pad | Verbindungskissen |
| 7 | Membrane | Membran |
| 8 | Absorbent Pad | Absorberkissen |

**Figur 2** Verhältnis zwischen "Bubble Point" und Porengröße
Der "Bubble Point" einer Membran ist der Druck, der benötigt wird, um die Luft durch eine nasse Membran zu pressen
**Figur 3**
Effekt der Kapillarflussrate auf die analytische Empfindlichkeit eines Lateral-Flow-Schnelltests

Beispiele:

Flussrate = 1,00 cm/min $\rightarrow$ effektive Analytenkonzentration = 1,00 x
Flussrate = 1,25 cm/min $\rightarrow$ effektive Analytenkonzentration = 0,65 x

**Figur 4**
Effekt einer Detergens- oder Netzmittelkonzentration auf verschiedene Leistungsmerkmale einer Membran

| 1 | Protein Binding | Proteinbindung |
|---|---|---|
| 2 | Capillary Flow Rate | Kapillare Flussrate |
| 3 | Striping Consistency | Streifenkonsistenz |
| 4 | Stripe Width | Streifenbreite |

**Figur 5**
Kalkulation der Bandbreite als Funktion der Verteilungsrate

Membranbettvolumenvolumen = $10\mu L/cm^2$
Reagentiendispensierrate = $1\mu L/cm$

$$\text{Bandbreite} = \frac{\text{Reagentiendispensierrate}}{\text{Membranbettvolumen}} = \frac{1\mu L/cm}{10\mu L/cm^2} = 0{,}1 \text{ cm}$$

**Figur 6**
Typische Zusammenhänge zwischen Flussrate einer Membran und Funktion eines immunchromatographischen Tests

| 1 | Surface Quality | Oberflächenqualität |
|---|---|---|
| 2 | Specificity | Spezifität |
| 3 | Sensitivity | Sensitivität |
| 4 | Total Assay Time | Gesamte Testzeit |

(fortgesetzt)

| 5 | Reagent Costs | Reagentienkosten |
|---|---|---|
| Sensitivität = analytische Empfindlichkeit = Nachweis in $\mu$g = Masse | | |

**Figuren 7-9**

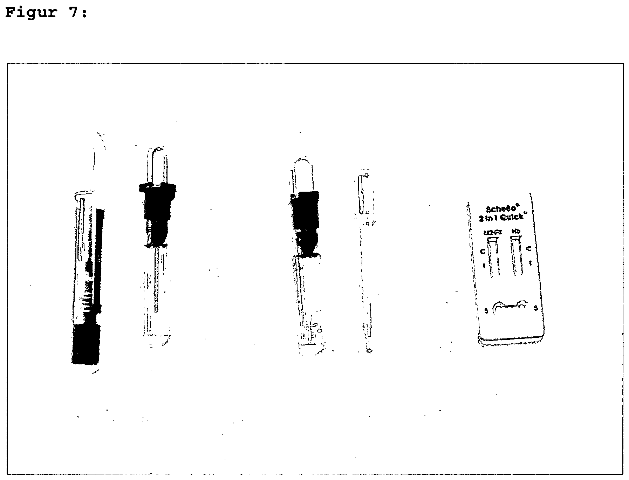

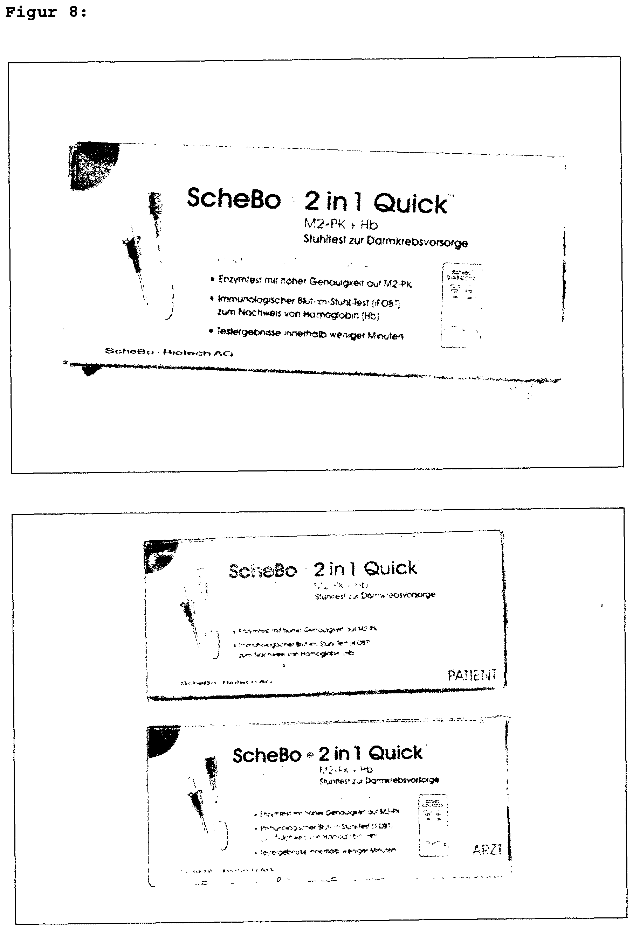

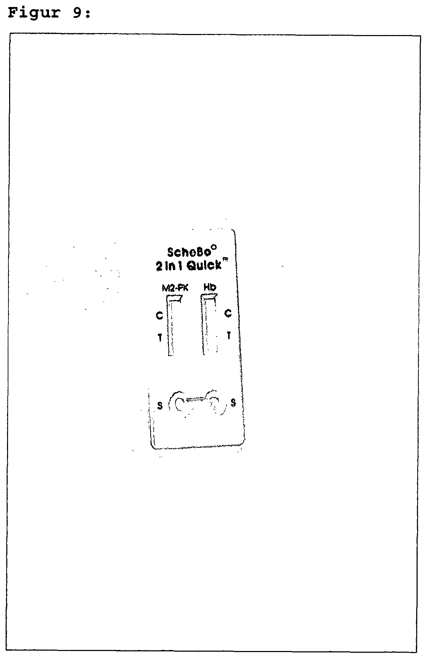

Die Figuren 7-9 zeigen in beispielhafter Form einen erfindungsgemäßen Testkit. In der Figur 7 sind die Probeentnahmevorrichtungen zu erkennen. Die Figur 9 zeigt die eigentliche Testkassette.

**Figur 10**

Figur 10 zeigt beispielhaft die Zuordnung der verschiedenen Messergebnisse zu Risikostufen. Zur Vereinfachung der Ablesung wird das Ergebnis vorteilhafterweise farbig dargestellt. Zur Vermeidung von Fehlablesungen von z.B. farbenblinden Personen können zusätzliche Unterscheidungsmerkmale angebracht werden (z.B. unterschiedliche Formen, Ziffern und/oder Buchstaben. Hierin beschrieben sind somit:

1. Hilfsmittel = Klassifikator zur Befundung (Diagnose) und Zuordnung in Wahrheitsmatrix von Befunden: richtige und falsche Klassifikationen. Da es sich um eine Ja/Nein Frage handelt, sagt man auch, der Test fällt *positiv* (Einordnung in auffällig) oder *negativ* (Einordnung nicht auffällig) aus. Fällt der Test für nur einen Biomarker positiv aus, so ist eine weitere Abklärung des positiven Befundes durch eine Koloskopie indiziert.

2. Hilfsmittel zur Klassifizierung, anhand bestimmter Merkmale (Bestimmung von M2-PK/Haemoglobin)

3. Hilfsmittel zur Entscheidung: soll Koloskopie zur weiteren Befundung durchgeführt werden: ja/nein

4. Hilfsmittel zur Erleichterung von ärztlichen Handlungsanweisungen durch verbesserte Einstiegsbefundung (erheblicher Zusatznutzen)

5. Hilfsmittel zur Erleichterung von ärztlichen Entscheidungen über Maßnahmen, z.B. Anwendung weiterer diagnostischer Verfahren z.B. Koloskopie usw. oder Therapiemaßnahmen, z.B. Chirurgie, Chemotherapie usw.

6. Hilfsmittel zur Verbesserung der Aussagegenauigkeit, Befundung auf Grund von Probandenstuhlproben

7. Testkit mit hoher Gesamt-Sensitivität und Gesamt-Spezifität für Hämoglobin und M2-PK zur Erkennung von Darmkrebs. Der Testkit dient als "Doppelvorfilter" im Rahmen des Darmkrebsvorsorgescreenings mittels Koloskopie.

8. Testkit mit geringer "Lot-to-Lot" Abweichung, die eine bestmögliche Vergleichbarkeit der Testergebnisse nicht nur innerhalb einer Herstellungscharge sondern auch zwischen den Chargen zulässt.

9. Hilfsmittel zur Vorhersage von Therapieerfolgen

10. Hilfsmittel zur Verbesserung des Therapiemonitorings

11. Hilfsmittel zur Visualisierung des Testergebnisses (Risikoampel)

12. Das erfindungsgemäße Testkit dient der vorausschauenden Problemvermeidung:

1. Der Prävention (lateinisch: praeveniere, zuvorkommen, verhüten) im Sinne vom Zuvorkommen unerwünschter Entwicklungen

a. gemäß Vermeidung von Krankheiten, von Krankheitsentstehung und Krankheitsausprägung (Schweregrad)
b. Vermeidung ärztlicher Handlungen z.B. unnötiger, gefährlicher, teurer Maßnahmen (Chirurgie, Chemotherapie, Nebenwirkungen)

Das erfindungsgemäße Testkit ist im Kontext der optimalen Schaden/Nutzen/Risiko¬Betrachtung im Vergleich auf die diagnostische Koloskopie zu sehen. Aber auch ganz wichtig ist die Erhaltung der Lebensqualität und ein Vermeiden eines frühzeitigen Sterbens.

13. Aufgabe des erfindungsgemäßen Testkits: Das Testkit dient zur Lösung eines dringenden Gesundheitsproblems weltweit. Das Testkit dient der "Auswahl" auffälliger Probanden (Effizienzsteigerung).

Dieses Hilfsmittel wird erfindungsgemäß durch die Bereitstellung eines Kombi-Schnelltests erreicht. Kombi-Schnelltest zur synchronen analytischen Bestimmung des Enzym-Biomarkers Tumor M2-PK und des Biomarkers Blut (Hämoglobin).
Kombi-Schnelltest enthaltend den Teststreifen (Tumor M2-PKplus) + den Teststreifen(iFOBplus) auf einer Testkassette
Kombi-Schnelltest zur synchronen analytischen Bestimmung des Enzym-Biomarkers Tumor M2-PK und des Biomarkers Hämo-Haptoglobin-Komplex (Hb-Hp-Komplex).
Kombi-Schnelltest enthaltend den Teststreifen (Tumor M2-PKplus) + den Teststreifen(Hb-Hp-Komplexplus) auf einer Testkassette.
Nachweisverfahren ist das immunchromatographische Verfahren. Beschrieben sind auch immunchemische Verfahren im array, miniarray-Format, auch turbidimetrische Verfahren möglich. Beschrieben sind auch monoklonale Antikörper Verwendung von besonderen Antikörpern im Kombi-Schnelltest, die sowohl spezifisch in ihren Bindungseigenschaften für Tumor M2-PK oder Hämoglobin sind und in immunchromatographischen Verfahren verwendbar sind (z.B. "membrantauglich", "detergenztauglich" sind).
Verwendung eines speziellen Antikörpers (Klon P1F3 AK erkennt spezifisch die räumliche, dimere Konformation der M2-PK) zur Bereitstellung des erfindungsgemäßen Kombi-Schnelltests. Dieser Tumor M2-PK-spezifische Antikörper bindet bevorzugt an eines der folgenden Epitope oder Fragmente von diesen oder Kombinationen (Epitope oder Fragmente von diesen) von diesen, die mindestens eine Länge von 4 Aminosäuren haben:

| | |
|---|---|
| LAPITSDP | EAEAAIYH |
| VEASFKCC | SGAIIVLT |
| CSGAIIVLT | LQLFEE |
| TEATAVGA | QLFEELRR |
| LRRLAPITSDPTEATA | VEASFKC |
| KCCSGAIIV | KSGRSAHG |

## Patentansprüche

1. Testkit zum Nachweis von Biomarkern im menschlichen Stuhl, bestehend aus
einem ersten Proberöhrchen, enthaltend eine erste Stuhlprobeentnahmevorrichtung zur Aufnahme der benötigten Stuhlmenge,
eine erste Pufferlösung, enthaltend 10-70 mM Hepespuffer mit pH-Wert 7,6-8,2,
einem zweiten Proberöhrchen, enthaltend eine zweite Stuhlprobeentnahmevorrichtung zur Aufnahme der benötigten Stuhlmenge,
eine zweite Pufferlösung, enthaltend 10-70 mM Acetatpuffer mit pH-Wert 5-6,
einer Testkassette
enthaltend ein Lateral-Flow Testsystem mit einer drei bis sechs Monate gelagerten Nitrozellulosemembran als stationärer Phase,
die Nitrozellulosemembran wiederum enthaltend monoklonaler Tumor M2-PK Antikörper der Maus, nämlich Klon PATAM3AT, IgG1 und Gold-gekoppelter monoklonaler MausAntikörper, nämlich Klon 1 E3, IgG1,
und monoklonaler Hämoglobin-Antikörper der Maus, nämlich Klon M1202100, IgG1, und Gold-gekoppelter monoklonaler Mausantikörper, nämlich Klon HB11-2312,
eine erste Öffnung zum Auftragen einer Stuhlprobe aus dem ersten Proberöhrchen,
eine zweite Öffnung zum Auftragen einer Stuhlprobe aus dem zweiten Proberöhrchen,
wobei das Analyseergebnis der ersten Stuhlprobe positiv ist, wenn der Gehalt an Tumor M2-PK größer ist als 4 $\pm$ 1 Units/ml Stuhlextrakt und das Analyseergebnis der zweiten Stuhlprobe positiv ist, wenn der Gehalt an Hämoglobin 24$\mu$g Hämoglobin pro Gramm Stuhl übersteigt.

2. Testkit gemäß Anspruch 1, **dadurch gekennzeichnet, dass** die Fängerantikörper an Goldkolloide gebunden sind.

3. Testkit gemäß Anspruch 1, **dadurch gekennzeichnet, dass** die vier möglichen Testergebnisse durch vier verschiedene Farben, Buchstaben, Ziffern, Zeichen und/oder geometrische Formen dargestellt werden.

4. Verwendung eines Testkits gemäß mindestens einem der Ansprüche 1-3 zur in-vitro Diagnostik einer pathologischen Veränderung des Darms.

5. Verfahren zur in-vitro Diagnostik eines malignen Geschehen im Gastrointestinaltrakt unter Verwendung eines Testkits gemäß mindestens eines der Ansprüche 1-3, **gekennzeichnet durch**
Entnahme von zwei Stuhlproben mittels zweier Entnahmevorrichtungen zur Aufnahme der jeweils benötigten Stuhlmenge,
Auflösung der ersten Stuhlprobe in der ersten Pufferlösung,
Auflösung der zweiten Stuhlprobe in der zweiten Pufferlösung,
Auftragung der ersten und der zweiten stuhlhaltigen Pufferlösung auf die jeweils zugeordnete Öffnung, Abwarten der vorgeschriebenen Wartezeit,
Ablesen der Ergebnisse in den jeweiligen Ableseöffnungen.

6. Verfahren gemäß Anspruch 5, **dadurch gekennzeichnet, dass** die Darstellung der 4 möglichen Testergebnisse folgendermaßen erfolgt:

| M2-PK | Hb | Ergebnis |
|-------|-----|------------------|
| - | - | Stufe 1 (grün) |
| - | + | Stufe 2 (gelb) |
| + | - | Stufe 3 (orange) |
| + | + | Stufe 4 (rot) |

**Claims**

1. A test kit for detecting biomarkers in human stool, consisting of
a first sample tube containing a first stool sampling device for receiving the required amount of stool,
a first buffer solution containing 10 - 70 mM of HEPES buffer with pH 7.6 to 8.2,
a second sample tube containing a second stool sampling device for receiving the required amount of stool,
a second buffer solution containing 10 - 70 mM of acetate buffer with pH 5 to 6,
a test cassette containing a lateral flow test system with a nitrocellulose membrane stored for three to six months as the stationary phase,
the nitrocellulose membrane, in turn, containing monoclonal tumor M2-PK mouse antibody, namely clone PATAM3AT, IgG1, and gold-coupled monoclonal mouse antibody, namely clone 1 E3, IgG1,
and monoclonal hemoglobin antibody of the mouse, namely clone M1202100, IgG1, and gold-coupled monoclonal mouse antibody, namely clone HB11-2312,
a first opening for applying a stool sample from the first sample tube,
a second opening for applying a stool sample from the second sample tube,
wherein the analysis result of the first stool sample is positive if the amount of tumor M2-PK is greater than $4 \pm 1$ units/ml of stool extract, and the analysis result of the second stool sample is positive, if the content of hemoglobin exceeds 24 $\mu$g hemoglobin per gram of stool.

2. A test kit according to claim 1, **characterized in that** the capture antibody is bound to gold colloids.

3. A test kit according to claim 1, **characterized in that** the four possible test results are represented by four different colors, letters, numbers, characters, and/or geometric shapes.

4. Use of a test kit according to at least one of claims 1 to 3 for *in vitro* diagnostics of a pathological change of the bowel.

5. A method for *in vitro* diagnostics of a malignant event in the gastrointestinal tract by using a test kit according to at least one of claims 1 to 3, **characterized by**
collecting two stool samples by means of using two sampling devices for receiving the respectively required amount of stool,
dissolving the first stool sample in the first buffer solution,

dissolving the second stool sample in the second buffer solution,
applying the first and second stool-containing buffer solutions on the respectively assigned opening,
waiting for the prescribed waiting period,
reading the results in the respective reading openings.

6. The method according to claim 5, **characterized by** that the representation of the four possible test results is made as follows:

| M2-PK | Hb | result |
|-------|-----|-----------------|
| - | - | Level 1 (green) |
| - | + | Level 2 (yellow) |
| + | - | Level 3 (orange) |
| + | + | Level 4 (red) |

**Revendications**

1. Kit de test pour détecter des biomarqueurs dans des selles humaines, consistant en
un premier tube à essai, contenant un premier dispositif de prise d'échantillons de selles pour recevoir la quantité requise de selles,
une première solution tampon, contenant 10 - 70 mM de tampon HEPES avec pH 7,6 à 8,2,
un deuxième tube à essai, contenant un deuxième dispositif de prise d'échantillons de selles pour recevoir la quantité requise de selles,
une deuxième solution tampon, contenant 10 - 70 mM de tampon acétate avec pH 5 à 6,
une cassette-test, contenant un système de test à «lateral flow» avec une membrane de nitrocellulose stockée pendant trois à six mois comme phase stationnaire,
la membrane de nitrocellulose, pour sa part, contenant
un anticorps monoclonal de souris de la tumeur M2-PK, c'est-à-dire clone PATAM3AT, IgG1, et un anticorps monoclonal de souris couplé par l'or, c'est-à-dire clone 1 E3, IgG1,
et un anticorps monoclonal hémoglobine de souris, c'est-à-dire clone M1202100, IgG1, et un anticorps monoclonal de souris couplé par l'or, c'est-à-dire clone HB11-2312,
une première ouverture pour appliquer un échantillon de selles à partir du premier tube à essai,
une deuxième ouverture pour appliquer un échantillon de selles à partir du deuxième tube à essai,
dans lequel le résultat de l'analyse du premier échantillon de selles est positif si la quantité de tumeur M2-PK est supérieure à 4 $\pm$ 1 unités/ml d'extrait de selles, et le résultat de l'analyse du deuxième échantillon de selles est positif, si le contenu en hémoglobine dépasse 24 μg hémoglobine par gramme de selles.

2. Kit de test selon la revendication 1, **caractérisé en ce que** l'anticorps capteur est lié à des colloïdes d'or.

3. Kit de test selon la revendication 1, **caractérisé en ce que** les quatre résultats de test possibles sont représentés par quatre couleurs, lettres, nombres, caractères et/ou formes géométriques différents.

4. Utilisation du kit de test selon au moins une des revendications 1 à 3 pour la diagnostique *in vitro* d'une altération pathologique de l'intestin.

5. Procédé de diagnostique *in vitro* d'un événement maligne dans le tractus gastro-intestinal en utilisant un kit de test selon au moins une des revendications 1 à 3, caractérisé en
prendre deux échantillons de selles au moyen de deux dispositifs de prise d'échantillons pour recevoir la quantité respectivement requise de selles,
dissoudre le premier échantillon de selles dans la première solution tampon,
dissoudre le deuxième échantillon de selles dans la deuxième solution tampon,
appliquer la première et la deuxième solution tampon contenant des selles sur l'ouverture respectivement associée,
attendre la période d'attente prescrite,
lire les résultats dans les ouvertures de lecture respectives.

**6.** Procédé selon la revendication 5, **caractérisé en ce que** la représentation des quatre résultats de test possibles se fait comme suit:

| M2-PK | Hb | résultat |
|:---:|:---:|:---:|
| - | - | Niveau 1 (vert) |
| - | + | Niveau 2 (jaune) |
| + | - | Niveau 3 (orange) |
| + | + | Niveau 4 (rouge) |

**Figur 1**

**Figur 2**

**Figur 3**

effektive
Analytenkonzentration

Flussrate (cm/min)

**Figur 4**

Optimale
Detergentienkonzentration

Detergentienkonzentration

**Figur 5**

Reagentienlinie

Minimumbreite= 0,1 cm

1 cm

**Figur 6**

1

2

3

4

5

langsam

Kapillarflussrate

schnell

Figur 7:

Figur 8:

Figur 9:

**Figur 10:**

| | Stufe 4 (rot) | 90% ige Wahrscheinlichkeit für fortgeschrittene Neoplasien. Möglichst umgehende Koloskopie. |
|---|---|---|
| M2-PK Hb<br>C ▬ ▬ C<br>T ▭ ▭ T<br>M2PK (+) Hb (+) | | |
| M2-PK Hb<br>C ▬ ▬ C<br>T ▭ T<br>M2PK (+) Hb (-) | Stufe 3 (orange) | Weitere Abklärung geboten (z.B. Koloskopie) |
| M2-PK Hb<br>C ▬ ▬ C<br>T T<br>M2PK (-) Hb (+) | Stufe 2 (gelb) | Weitere Abklärung geboten (z.B. Koloskopie) |
| M2-PK Hb<br>C ▬ ▬ C<br>T T<br>M2PK (-) Hb (-) | Stufe 1 (grün) | Darmkrebs kann mit >97%iger Sicherheit ausgeschlossen werden |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0009303 W
- WO 2007071366 A1
- US 433734 A
- US 4376110 A
- US 4435504 A
- US 4703017 A
- US 4855240 A
- US 4954452 A
- US 5028535 A
- US 5075078 A
- US 9516207 B
- US 5654162 A
- EP 0810436 A1
- WO 0121826 A2
- WO 0250546 A2
- WO 03069343 A2
- WO 03065003 A2
- WO 0121826 A
- US 5270163 A
- DE 10205709 A1

### In der Beschreibung aufgeführte Nicht-Patentliteratur

- *Anticancer Research,* 1999, vol. 19, 2785-2820
- **LEMAN ES et al.** *Cancer Res.,* 2007, vol. 67 (12), 5600-5605
- **LEMAN ES et al.** *Clinical Cancer Research,* 2008, vol. 14, 1349-1354
- **HERMANN BRENNER et al.** *Int. J. Cancer,* 2010
- **H. BRENNER ; S. TAO.** *European Journal of Cancer,* 2013
- **BRENNER et al.** *Int. J. Cancer,* 2010
- **FAVENNE L. et al.** *Ann. Biol. Clin.,* 1992, vol. 50, 311-313
- **THOMAS L.** Labor und Diagnose. 1998
- **KOEHLER ; MILSTEIN.** *Nature,* 1995, vol. 256, 495-497
- **SUMEDHA.** *Clin. Chem.,* 1999, vol. 45, 1628-1650